# EUROPÄISCHE PATENTSCHRIFT

(11) **EP 0 600 836 B1**
(45) Veröffentlichungstag und Bekanntmachung des Hinweises auf die Patenterteilung: **05.08.1998**
(21) Anmeldenummer: 93810810.7
(22) Anmeldetag: 23.11.1993
(51) Int. Cl.: A01N 47/36, C07D 213/75

(54) **Selektiv-herbizides Mittel**
Selective herbicidal agent
Agent selectivement herbicide

(30) Priorität: 02.12.1992 CH 3697/92; 25.01.1993 CH 213/93; 10.02.1993 CH 397/93; 25.03.1993 CH 906/93
(43) Veröffentlichungstag der Anmeldung: 08.06.1994
(73) Patentinhaber: Novartis AG, 4058 Basel (CH)
(72) Erfinder: Föry, Werner, Dr., CH-4125 Riehen (CH); Kerber, Elmar, Dr., D-79733 Görwihl (DE); Hudetz, Manfred, Dr., CH-4310 Rheinfelden (CH)

(56) Entgegenhaltungen:
- EP-A- 0 103 543
- EP-A- 0 365 484
- EP-A- 0 459 949
- EP-A- 0 492 367
- EP-A- 0 555 770
- WO-A-92/16522
- WO-A-93/17016

## Beschreibung

Die vorliegende Erfindung betrifft ein selektiv-herbizides Mittel zur Bekämpfung von Gräsern und Unkräutern in Kulturen von Nutzpflanzen, insbesondere in Kulturen von Mais und Zuckerrohr, welches ein Herbizid und einen Safener (Gegenmittel, Antidot) enthält, welches die Nutzpflanzen, nicht aber die Unkräuter vor der phytotoxischen Wirkung des Herbizides bewahrt, sowie die Verwendung dieses Mittels oder der Kombination Herbizid und Safener zur Unkrautbekämpfung in Nutzpflanzenkulturen.

Beim Einsatz von Herbiziden können in Abhängigkeit von Faktoren wie beispielsweise Dosis des Herbizids und Applikationsart, Art der Kulturpflanze, Bodenbeschaffenheit und klimatischen Bedingungen, wie beispielsweise Belichtungsdauer, Temperatur und Niederschlagsmengen, die Kulturpflanzen in erheblichem Maße geschädigt werden. Insbesondere kann es zu starken Schädigungen kommen, wenn im Rahmen der Fruchtfolge nach Kulturpflanzen, die gegen die Herbizide resistent sind, andere Kulturpflanzen angebaut werden, welche keine oder nur unzureichende Resistenz gegenüber den Herbiziden aufweisen.

Um diesem Problem zu begegnen, sind schon verschiedene Stoffe vorgeschlagen worden, welche befähigt sind, die schädigende Wirkung des Herbizids auf die Kulturpflanze spezifisch zu antagonisieren, das heißt, die Kulturpflanze zu schützen ohne dabei die Herbizidwirkung auf die zu bekämpfenden Unkräuter merklich zu beeinflussen. Dabei hat es sich gezeigt, daß die vorgeschlagenen Safener sowohl bezüglich der Kulturpflanzen als auch bezüglich des Herbizids und gegebenenfalls auch in Abhängigkeit von der Applikationsart oft sehr artspezifisch wirken, das heißt ein bestimmter Safener eignet sich oft nur für eine bestimmte Kulturpflanze und eine spezielle Herbizid-Stoffklasse.

Beispielsweise eignen sich aus EP-A-0 365 484 bekannte Sulfamoylphenylharnstoffe zum Schützen von Kulturpflanzen vor der phytotoxischen Wirkung einer ganz speziellen Klasse von Pyridylsulfonylharnstoffherbiziden.

Erfindungsgemäß wird somit ein selektiv-herbizides Mittel vorgeschlagen, welches dadurch gekennzeichnet ist, daß es neben inerten Träger- und Zusatzstoffen als wirksame Komponente eine Mischung
a) aus einer herbizid wirksamen Menge eines Pyridylsulfonylharnstoffes der Formel I 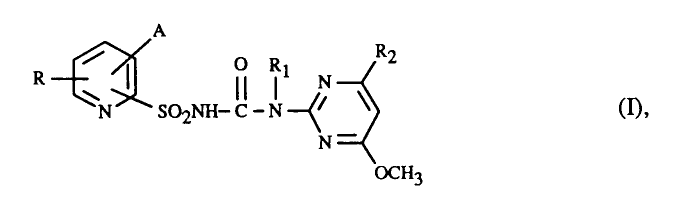 worin
   R Wasserstoff, Halogen, C₁-C₄-Alkyl, C₁-C₄-Alkoxy oder C₁-C₄-Alkylthio, oder durch Halogen ein- oder mehrfach substituiertes C₁-C₄-Alkyl;
   R₁ Wasserstoff oder Methyl;
   R₂ Methyl oder Methoxy;
   A-X-R₃ oder-N-(R₄)R₅;
   R₃ C₁-C₆-Alkyl, oder C₁-C₆-Alkyl welches ein- oder mehrfach durch Halogen, C₁-C₆-Alkoxy, C₃-C₆-Alkenyloxy, C₃-C₆-Alkinyloxy oder C₃-C₆-Cycloalkyl, welches seinerseits durch Sauerstoff unterbrochen sein kann, substituiert ist, C₃-C₆-Alkenyl, oder C₃-C₆-Alkenyl welches ein- oder mehrfach durch Halogen, C₁-C₆-Alkoxy, C₃-C₆-Alkenyloxy oder C₃-C₆-Alkinyloxy substituiert ist, C₄-C₆-Cycloalkyl, oder C₄-C₆-Cycloalkyl welches ein- oder mehrfach durch Halogen oder C₁-C₆-Alkoxy substituiert ist, oder C₃-C₆-Cycloalkyl, welches durch Sauerstoff unterbrochen ist, oder C₃-C₆-Alkinyl;
   X Sauerstoff oder S(O)ₙ;
   n 0, 1 oder 2;
   R₄ Wasserstoff, C₁-C₆-Alkyl, oder C₁-C₆-Alkyl welches ein- oder mehrfach durch Halogen, C₁-C₆-Alkoxy, C₃-C₆-Alkenyloxy, C₃-C₆-Alkinyloxy oder C₁-C₆-Alkylthio substituiert ist;
   R₅ Wasserstoff, C₁-C₆-Alkyl, oder C₁-C₆-Alkyl welches ein- oder mehrfach durch Halogen, C₁-C₆-Alkoxy, C₃-C₆-Alkenyloxy, C₃-C₆-Alkinyloxy oder C₁-C₆-Alkylthio substituiert ist, oder C(O)R₆; und
   R₆ Wasserstoff, C₁-C₆-Alkyl, C₃-C₆-Cycloalkyl, C₂-C₆-Alkinyl; oder C₁-C₆-Alkyl oder C₃-C₆-Cycloalkyl substituiert durch Halogen oder C₁-C₄-Alkoxy; C₂-C₆-Alkenyl oder C₂-C₆-Alkenyl substituiert durch Halogen; Phenyl, Benzyl, Naphtyl oder OR₁₂, oder Phenyl, Benzyl, Naphtyl substituiert durch Halogen, C₁-C₄-Alkyl, C₁-C₄-Halogenalkyl, C₁-C₄-Alkoxy, C₁-C₄-Halogenalkoxy, C₃-C₆-Alkenyloxy, C₃-C₆-Alkinyloxy, Nitro, Cyano, COOR₁₃, NR₁₅R₁₆, C(O)NR₁₇R₁₈, X₁R₂₀, SO₂NR₂₁R₂₂ oder X₂R₂₃;
   R₁₂ C₁-C₆-Alkyl, C₁-C₆-Halogenalkyl, Oxetan-3-yl, C₄-C₆-Cycloalkyl, welches seinerseits durch Halogen, C₁-C₄-Alkyl oder C₁-C₄-Alkoxy substituiert sein kann; Phenyl, Benzyl, Naphtyl oder Phenyl, Benzyl, Naphtyl substituiert durch C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfonyl, C₁-C₄-Alkylsulfinyl, Nitro, Cyano, COOR₂₇, NR₂₅R₂₆, CONR₂₈R₂₉ oder SO₂NR₃₀R₁₄; C₁-C₆-Alkyl substituiert durch C₁-C₄-Alkoxy, C₃-C₆-Cycloalkyl, Cyano, COOR₂₄ oder CONR₃₂R₃₃; C₃-C₆-Alkenyl, C₃-C₆-Halogenalkenyl, C₃-C₆-Alkinyl, C₃-C₆Halogenalkinyl, X₃R₃₅ oder X₄R₃₆;
   R₁₃ Wasserstoff, C₁-C₆-Alkyl, C₃-C₆-Alkenyl, C₃-C₆-Alkinyl oder Oxetan-3-yl;
   R₁₄, R₁₅, R₁₆, R₁₇, R₁₈, R₂₁, R₂₂, R₂₅, R₂₆, R₂₈, R₂₉, R₃₀, R₃₂ und R₃₃ unabhängig voneinander Wasserstoff, C₁-C₄-Alkyl, C₃-C₆-Alkenyl oder C₃-C₆-Alkinyl; oder R₁₅ und R₂₅ unabhängig voneinander die Gruppen -C(O)-X₅-C₁-C₄-Alkyl oder -C(O)-C₁-C₄-Alkyl, welche ihrerseits durch Halogen substituiert sein können;
   oder R₁₅ und R₁₆ oder R₁₇ und R₁₈ oder R₂₁ und R₂₂ oder R₂₅ und R₂₆ oder R₂₈ und R₂₉ oder R₃₀ und R₁₄ oder R₃₂ und R₃₃ zusammen für eine C₄-C₅-Alkylenkette, die seinerseits durch Sauerstoff oder NR₁₉ unterbrochen sein kann,
   R₁₉ Wasserstoff, C₁-C₄-Alkyl, C₃-C₆-Alkenyl oder C₃-C₆-Alkinyl;
   R₂₀ und R₃₅ unabhängig voneinander C₁-C₄-Alkyl oder C₁-C₄-Halogenalkyl;
   R₂₃ und R₃₆ unabhängig voneinander C₁-C₄-Alkyl substituiert durch COOR₃₄;
   R₂₄, R₂₇ und R₃₄ unabhängig voneinander Wasserstoff oder C₁-C₄-Alkyl;
   X₁ und X₃ unabhängig voneinander Schwefel, SO oder SO₂;
   X₂ und X₄ unabhängig voneinander Sauerstoff oder Schwefel; X₅ Sauerstoff oder NR₃₇; und
   R₃₇ Wasserstoff, C₁-C₄-Alkyl, C₃-C₆-Alkenyl oder C₃-C₆-Alkinyl bedeutet;
      oder einem N-Oxid oder einem Salz der Verbindung der Formel I, und
b) aus einer herbizid-antagonistisch wirksamen Menge eines Sulfamoylphenylharnstoffes der Formel II 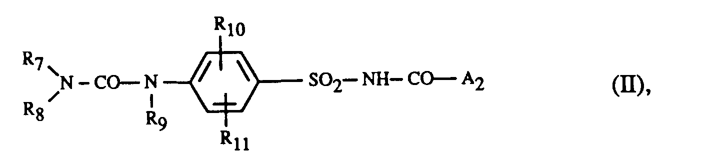 worin
   A₂ für einen Rest aus der Gruppe 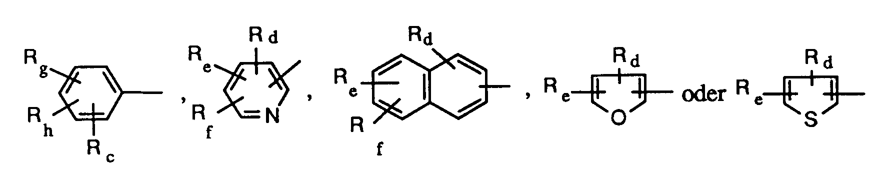 ist,
   R₇ und R₈ unabhänig voneinander für Wasserstoff, C₁-C₈-Alkyl, C₃-C₈-Cycloalkyl, C₃-C₆-Alkenyl, C₃-C₆-Alkinyl, 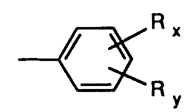 oder durch C₁-C₄-Alkoxy oder 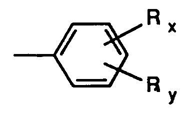 substituiertes C₁-C₄-Alkyl, oder
   R₇ und R₈ gemeinsam für eine C₄-C₆-Alkylenbrücke oder eine durch Sauerstoff, Schwefel, SO, SO2, NH oder -N(C₁-C₄-Alkyl)- unterbrochene C₄-C₆-Alkenylenbrücke stehen;
   R₉ Wasserstoff oder C₁-C₄-Alkyl ist;
   R₁₀ und R₁₁ unabhängig voneinander für Wasserstoff, Halogen, Cyano, Nitro, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl,-COORⱼ, CONRₖRₘ,-CORₙ,-SO₂NRₖRₘ oder-OSO₂-C₁-C₄-Alkyl; oder R₁₀ und R₁₁ gemeinsam für eine C₃-C₄-Alkylenbrücke, die durch Halogen oder C₁-C₄-Alkyl substituiert Sein kann, oder eine C₃-C₄-Alkenylenbrücke, die durch Halogen oder C₃ oder C4-Alkyl substituiert sein kann, oder eine Butadienylenbrücke, die durch Halogen oder C₁-C₄-Alkyl substituiert sein kann, und
   R_{g} und Rₕ unabhängig voneinander für Wasserstoff, Halogen, C₁-C₄-Alkyl, Trifluormethyl, Methoxy, Methylthio oder -COORⱼ stehen; wobei
   R_{c} Wasserstoff, Halogen, C₁-C₄-Alkyl oder Methoxy;
   R_{d} Wasserstoff, Halogen, Nitro, C₁-C₄-Alkyl, C₁-C₄-Alkoxy, C₁-C₄-Alkylthio, C₁-C₄-Alkylsulfinyl, C₁-C₄-Alkylsulfonyl, -COORⱼ oder -CONRₖRₘ;
   Rₑ Wasserstoff, Halogen, C₁-C₄₄-Halogenalkyl, C₁-C₄-Alkyl, -COORⱼ, Trifluromethyl oder Methoxy bedeuten; oder
   R_{d} und Rₑ gemeinsam für eine C₃-C₄-Alkylenbrücke stehen;
   R_{f} Wasserstoff, Halogen oder C₁-C₄-Alkyl ist;
   Rₓ und R_{y} unabhängig voneinander Wasserstoff, Halogen, C₁-C₄-Alkyl, C₁-C₄-Alkoxy, C₁-C₄-Alkylthio, -COOR₁₇, Trifluormethyl, Nitro oder Cyano ist;
   R₁₇ für Wasserstoff, C₁-C₁₀-Alkyl, C₁-C₄-Alkoxy-C₁-C₄-alkyl, C₁-C₄-Alkylthio-C₁-C₄-alkyl, Di-C₁-C₄-alkylamino-C₁-C₄-allcyl, _{H}alogen-C₁-C₈-alkyl, C₂-C₈-Alkenyl, Halogen-C₂-C₈-alkenyl, C₃-C₈-Alkinyl, C₃-C₇-Cycloalkyl, Halogen-C₃-C₇-cycloalkyl, C₁-C₈-Alkylcarbonyl, Allylcarbonyl, C₃-C₇-Cycloalkylcarbonyl, Benzoyl, das unsubstituiert oder am Phenylring gleich oder verschieden bis zu dreifach durch Halogen, C₁-C₄-Alkyl, Halogen-C₁-C₄-alkyl, Halogen-C₁-C₄-alkoxy oder C₁-C₄-Alkoxy substituiert ist; oder Furoyl, Thienyl; oder C₁-C₄-Alkyl substituiert durch Phenyl, Halogenphenyl, C₁-C₄-Alkylphenyl, C₁-C₄-Alkoxyphenyl, Halogen-C₁-C₄-alkylphenyl, Halogen-C₁-C₄-alkoxyphenyl, C₁-C₆-Alkoxycarbonyl, C₁-C₄-Alkoxy-C₁-C₈-alkoxycarbonyl, C₃-C₈-Alkenyloxycarbonyl, C₃-C₈-Alkinyloxycarbonyl, C₁-C₈-Alkylthiocarbonyl, C₃-C₈-Alkenylthiocarbonyl, C₃-C₈-Alkinylthiocarbonyl, Carbamoyl, Mono-C₁-C₄-alkylaminocarbonyl, Di-C₁-C₄-alkylaminocarbonyl; oder Phenylaminocarbonyl, das unsubstituiert oder am Phenyl gleich oder verschieden bis zu dreifach durch Halogen, C₁-C₄-Alkyl, Halogen-C₁-C₄-alkyl, Halogen-C₁-C₄-alkoxy oder C₁-C₄-Alkoxy oder einfach durch Cyano oder Nitro substituiert ist, oder Dioxolan-2-yl, das unsubstituiert ist oder durch ein oder zwei C₁-C₄-Alkylreste substituiert ist, oder Dioxan-2-yl, das unsubstituiert ist oder durch ein oder zwei C₁-C₄-Alkylreste substituiert ist, oder C₁-C₄-Alkyl, das durch Cyano, Nitro, Carboxyl oder C₁-C₈-Alkylthio-C₁-C₈-alkoxycarbonyl substituiert ist, steht;
   Rⱼ, Rₖ und Rₘ unabhängig voneinander Wasserstoff oder C₁-C₄-Alkyl ist;
   Rₖ und Rₘ gemeinsam eine C₄-C₆-Alkylenbrücke oder eine durch Sauerstoff, NH oder -N(C₁-C₄-Alkyl)- unterbrochene C₄-C₆-Alkylenbrücke bilden; und
   Rₙ C₁-C₄-Alkyl, Phenyl oder durch Halogen, C₁-C₄-Alkyl, Methoxy, Nitro oder Trifluormethyl substituiertes Phenyl ist,
      oder einem Salz der Verbindung der Formel II, enthält.

Gegenstand der vorliegenden Erfindung ist ferner die Verwendung des erfindungsgemäßen Mittels zur Bekämpfung von Unkräutern und Gräsern in Nutzpflanzenkulturen, insbesondere Mais und Zuckerrohr.

Unter Halogen ist in den Definitionen Fluor, Chlor, Brom und Jod, vorzugsweise jedoch Fluor, Chlor und Brom, insbesondere Chlor zu verstehen. Unter Alkyl ist geradkettiges oder verzweigtes Alkyl zu verstehen; zum Beispiel Methyl, Aethyl, n-Propyl, i-Propyl oder die vier isomeren Butyl. Längerkettige Alkylgruppen umfassen die Isomeren von Pentyl, Hexyl, Heptyl oder Oktyl, wobei jeweils die unverzweigten Ketten bevorzugt sind. Unter Alkoxy ist zu verstehen: Methoxy, Aethoxy, n-Propyloxy, i-Propyloxy oder die vier isomeren Butyloxy-, Pentyloxy- und Hexyloxyreste, insbesondere aber Methoxy, Ethoxy oder i-Propyloxy. Durch Alkoxy substituiertes Alkyl steht vorzugsweise für Methoxymethyl, Aethoxymethyl, Methoxyethyl und Ethoxyethyl, insbesondere aber Methoxyethyl. Durch gegebenenfalls substituiertes Phenyl substituiertes Alkyl steht vorzugsweise für Abkömmlinge von Phenylethyl oder Benzyl. Typische Alkenyl-und Alkinylreste sind Allyl, 2-Butenyl, Methallyl, 3-Butenyl, Propargyl, 2-Butinyl, 3-Butinyl oder 2-Pentenyl. Beispiele für Cycloalkyl sind Cyclopropyl, Cyclopentyl, Cyclohexyl oder Cycloheptyl, vorzugsweise aber Cyclopentyl und Cyclohexyl. Heterocyclen sind z.B. Pyrrolidin, Piperidin, Pyrazolidin, Imidazolidin, Oxazolidin, Thiazolidin, Morpholin, Thiomorpholin, Piperazin oder Hexahydroazepin sowie bei schwefelhaltigen Ringen deren Oxidationsprodukte. Durch Sauerstoff unterbrochenes C₃-C₆-Cycloalkyl ist beispielsweise Oxetan, Oxolan, Oxan, Oxepan oder Dioxan. In Alkylthio, Alkylsulfinyl oder Alkylsulfonyl hat Alkyl die oben aufgezählten konkreten Bedeutungen.

Wenn Substituenten gemeinsam für eine C₃-C₄-Alkylenbrücke, C₃-C₄-Alkenylenbrücke oder Butadienylenbrücke, die jeweils durch Halogen oder C₁-C₄-Alkyl substituiert sein kann, stehen, werden z.B. zusammen mit einem Phenylring, an welchem die Brücke gebunden ist, zweikernige Systeme gebildet wie beispielsweise 1,2,3,4-Tetrahydro-naphtatin, 1-Chlor-2-methyl-3,4-dihydronaphtalin, Indan, 1,2-Dihydronaphtalin, Inden, Naphtalin, 2-Methylnaphtalin, 1-n-Butylnaphtalin, 2-Ethylnaphtalin oder 1-Chlornaphtalin.

Wenn Substituenten gemeinsam für eine C₃ oder C₄-Alkylenbrücke stehen, so werden zusammen mit dem Ringsystem, an welches sie gebunden sind, mehrkernige Systeme gebildet wie etwa 2,3-Tetramethylenthiophen, 2,3-Trimethylenthiophen, 2,3-Tetramethylenfuran, 3,4-Tetramethylenpyridin oder

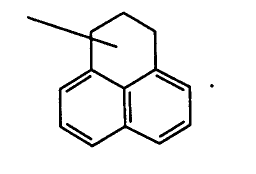

Unter N-Oxiden der Verbindung der Formel I sind Verbindungen zu verstehen, die eine Gruppe der Formel 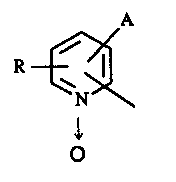 enthalten.

Die Erfindung umfaßt ebenfalls die Salze, die die Verbindungen der Formel I und II mit Aminen, Alkali- und Erdalkalimetallbasen oder quaternären Ammoniumbasen bilden können. Ebenso kann Salzbildung durch Anlagerung einer starken Säure an den Pyrimidinteil der Verbindung der Formel I erfolgen. Geeignete Säuren hierfür sind Salzsäure, Bromwasserstoffsäure, Schwefelsäure oder Salpetersäure.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Als Beispiele für zur Ammonium-Kationenbildung geeignete Amine kommen sowohl Ammoniak wie auch primäre, sekundäre und teriäre C₁-C₄-Alkylamin, C₁-C₄-Hydroxyalkylamine und C₂-C₄-Alkoxyalkylamine in Betracht, beispielsweise Methylamin, Ethylamin, n-Propylamin, iso-Propylamin, die vier isomeren Butylamine, n-Amylamin, iso-Amylamin, Hexylamin, Heptylamin, Octylamin, Nonylamin, Decylamin, Pentadecylamin, Hexadecylamin, Heptadecylamin, Octadecylamin, Methyl-ethylamin, Methyl-iso-propylamin, Methyl-hexylamin, Methyl-nonylamin, Methyl-pentadecylamin, Methyl-octadecylamin, Ethyl-butylamin, Ethyl-heptylamin, Ethyl-octylamin, Hexyl-heptylamin, Hexyl-octylamin, Dimethylamin, Diethylamin, Di-n-propylamin, Di-iso-propylamin, Di-n-butylamin, Di-n-amylamin, Di-iso-amylamin, Dihexylamin, Di-heptylamin, Dioctylamin, Ethanolamin, n-Propanolamin, iso-Propanolamin, N,N-Di-N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propylendiamin, Diethanolamin, Trimethylamin, Triethylamin, Tri-n-propylamin, Tri-iso-propylamin, Tri-n-butylamin, Tri-iso-butylamin, Tri-sek.-butylamin, Tri-n-amylamin, Methoxyethylamin und Ethoxyethylamin; heterocyclische Amine wie z.B. Pyridin, Chinolin, iso-Chinolin, Morpholin, Piperidin, Pyrrolidin, Indolin, Chinuclidin und Azepin; primäre Arylamine wie z.B. aniline, Methoxyaniline, Ethoxyaniline, o,m,p-Toluidine, Phenylendiamine, Benzidine, Naphthylamine und o,m,p-Chloraniline; insbesondere aber Triethylamin, iso-Propylamin und Di-iso-propylamin.

Beispiele für quarternäre Ammoniumbasen sind im allgemeinen die Kationen von Halogenammoniumsalzen, z.B. das Tetramethylammoniumkation, das Trimethylbenzylammoniumkation, das Triethylbenzylammoniumkation, das Tetraethylammoniumkation, das Trimethylethylammoniumkation, aber auch das Ammoniumkation.

Vorzugsweise steht R₆ für Wasserstoff, C₁-C₆-Alkyl, C₃-C₆-Cycloalkyl, C₂-C₆-Alkinyl; oder C₁-C₆-Aikyl oder C₃-C₆-Cycloalkyl substituiert durch Halogen oder C₁-C₄-Alkoxy; C₂-C₆-Alkenyl oder C₂-C₆-Alkenyl substituiert durch Halogen.

In bevorzugten erfindungsgemäßen Mitteln steht in der Formel I R₆ für Wasserstoff, C₁-C₆-Alkyl oder C₃-C₆-Cycloalkyl, besonders bevorzugt für Wasserstoff oder C₁-C₆-Alkyl. Ferner sind erfindungsgemäße Mittel bevorzugt, bei denen Verbindungen der Formel I verwendet werden, worin R₃ für C₁-C₆-Alkyl oder C₁-C₆-Alkyl welches ein-oder mehrfach durch Halogen, C₁-C₆-Alkoxy, C₃-C₆-Alkenyloxy, C₃-C₆-Alkinyloxy oder C₃-C₆-Cycloalkyl, welches seinerseits durch Sauerstoff unterbrochen sein kann, substituiert ist, C₃-C₆-Alkenyl, oder C₃-C₆-Alkenyl welches ein- oder mehrfach durch Halogen, C₁-C₆-Alkoxy, C₃-C₆-Alkenyloxy oder C₃-C₆-Alkinyloxy substituiert ist, C₄-C₆-Cycloalkyl, oder C₄-C₆-Cycloalkyl welches ein- oder mehrfach durch Halogen oder C₁-C₆-Alkoxy substituiert ist oder durch Sauerstoff unterbrochen sein kann, oder C₃-C₆-Alkinyl steht und R₆ Wasserstoff oder C₁-C₆-Alkyl bedeutet.

Für die Verwendung im erfindungsgemäßen Mittel bevorzugte Verbindungen der Formel I sind ferner solche, worin der Pyridylsulfonylharnstoff der Formel Ia 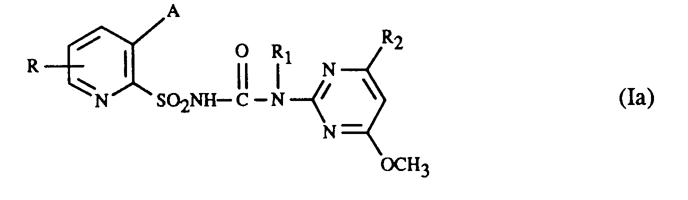 entspricht, worin A, R, R₁ und R₂ die unter Formel I angegebenen Bedeutungen haben.

Erfindungsgemäße Mittel, die diese Verbindung der Formel Ia zusammen mit einer Verbindung der Formel IIf 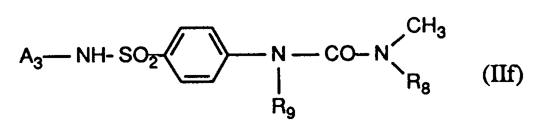 worin R₈ und R₉ die unter Formel II angegebenen Bedeutung haben und A₃ für die Gruppen 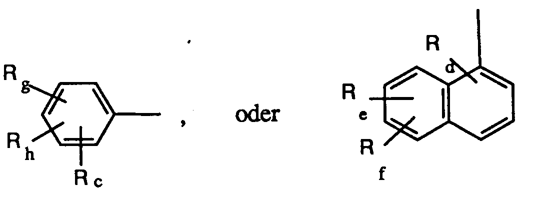 oder steht, sind besonders bevorzugt, wobei in der Formel IIf R₈ und R₉ insbesondere für Wasserstoff oder Methyl stehen.

Von diesen Mitteln sind diejenigen besonders bevorzugt, in denen
a) R Wasserstoff bedeutet; oder
b) A -X-R₃ bedeutet; oder
c) A -N-(R₄)R₅ bedeutet; wobei R₅ vorzugsweise für C(O)R₆ steht.

Ferner sind diejenigen erfindungsgemäßen Mittel von Interesse, in denen ein Herbizid der Formel Ia verwendet wird, worin R und R₁ Wasserstoff und A Difluormethoxy oder die Gruppe 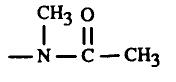 bedeuten.

Besonders herausragende Mittel enthalten eine Verbindung der Formel Ia, worin R und R₁ Wasserstoff und A die Gruppe 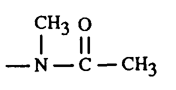 oder -SO₂-C₁-C₄-Alkyl bedeuten.

Für die Verwendung im erfindungsgemäßen Mittel bevorzugte Verbindungen der Formel II sind dadurch gekennzeichnet, daß A₂ ein Rest der Formel 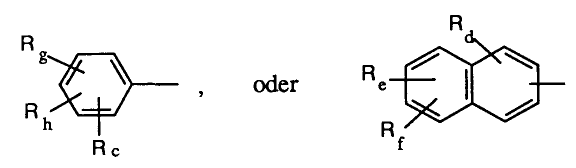 oder ist, worin
R_{g} und Rₕ unabhängig voneinander für Wasserstoff, Halogen, C₁-C₄-Alkyl, Trifluormethyl, Methoxy, Methylthio oder COORⱼ stehen; wobei
R_{c} Wasserstoff, Halogen, C₁-C₄-Alkyl oder Methoxy;
R_{d} Wasserstoff, Halogen, Nitro, C₁-C₄-Alkyl, C₁-C₄-Alkoxy, C₁-C₄-Alkylthio, C₁-C₄-Alkylsulfinyl, C₁-C₄-Alkylsulfonyl, -COORⱼ oder -CONRₖRₘ;
Rₑ Wasserstoff, Halogen, C₁-C₄-Alkyl, -COORⱼ, Trifluormethyl oder Methoxy ist; oder R_{d} und Rₑ gemeinsam für eine C₃ oder C₄-Alkylenbrücke bilden;
R_{f} Wasserstoff, Halogen oder C₁-C₄-Alkyl ist;
Rⱼ, Rₖ und Rₘ unabhängig voneinander Wasserstoff oder C₁-C₄-Alkyl sind; und
Rₖ und Rₘ gemeinsam eine C₄-C₆-Alkylenbrücke oder eine durch Sauerstoff, NH oder -N(C₁-C₄-Alkyl)- unterbrochene C₄-C₆-Alkylenbrücke bilden.

Hiervon sind solche besonders bevorzugt, worin R_{c}, R_{d}, Rₑ, R_{g} und Rₕ unabhängig voneinander Wasserstoff, C₁-C₄-Alkyl oder Methoxy sind.

Weitere besonders geeignete Gruppen von Verbindungen der Formel II sind solche, worin R₁₀ und R₁₁ oder R₈ und R₉ Wasserstoff bedeuten.

Besonders hervorzuheben sind auch diejenigen Verbindungen der Formel II, worin R₉, R₁₀ und R₁₁ Wasserstoff sind.

Besonders wertvolle Mittel gemäß vorliegender Erfindung zeichnen sich dadurch aus, daß sie als Verbindung der Formel I eine Verbindung der Formel Ia 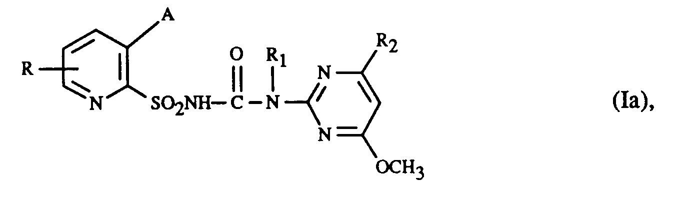 worin A, R, R₁, R₂ und R₃ die unter Formel I angegebenen Bedeutungen haben; und als Verbindung der Formel II eine Verbindung der Formel IIa 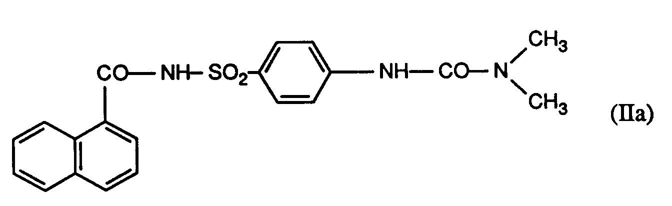 oder der Formel IIb 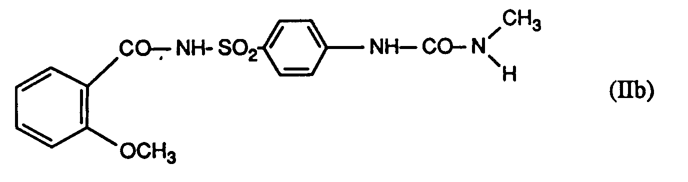 oder der Formel IIc 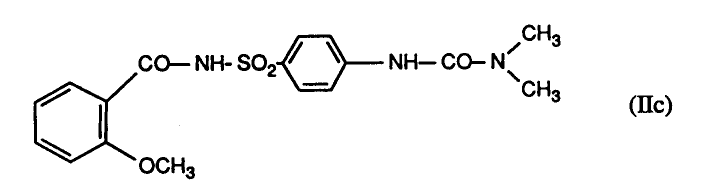 oder der Formel IId 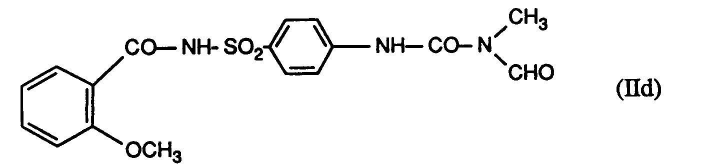 oder der Formel IIe 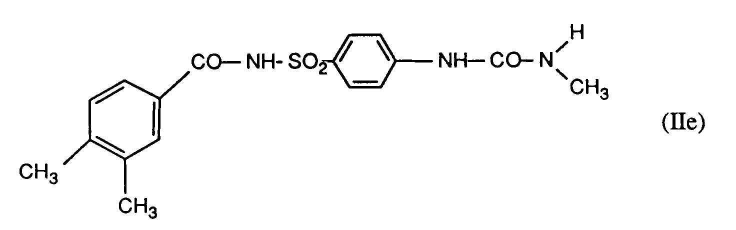 enthalten.

Die erfindungsgemäß verwendeten Pyridylsulfonylharnstoffe der Formel I sind entweder bekannt oder lassen sich analog bekannter Methoden herstellen. Die Herstellung derartiger Verbindungen ist beispielsweise in EP-A-0 103 543, EP-A-0 459 949, EP-A-0 555 770 und WO 92/16522 beschrieben. Die für die erfindungsgemäßen Mittel verwendeten Sulfamoylphenylharnstoffe der Formel II und ihre Herstellung sind z.B. aus der EP-A-0 365 484 bekannt.

Weitere, für die Verwendung im im erfindungsgemäßen Mittel besonders gut geeignete, herbizid wirksame Pyridylsulfonylharnstoffe entsprechen der Formel Ib 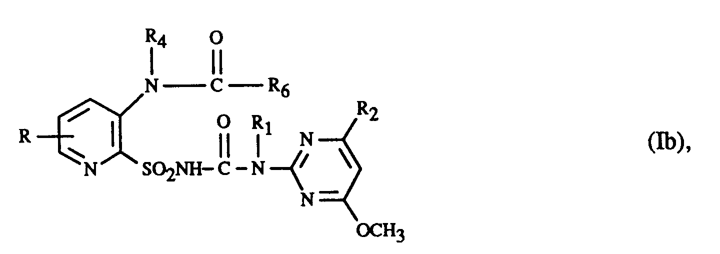 worin R, R₁, R₂, R₄ und R₆ die unter Formel I angegebene Bedeutung haben. Von diesen Verbindungen der Formel Ib sind diejenigen bevorzugt, bei denen R₆ Wasserstoff oder C₁-C₆-Alkyl bedeutet.

Ferner sind diejenigen Verbindungen der Formel Ib bevorzugt, bei denen R und R₁ Wasserstoff bedeuten. Aus dieser Gruppe sind Verbindungen von besonderem Interesse, bei denen R₄ und R₆ Methyl bedeuten.

Die Verbindungen der Formel Ib können hergestellt werden, indem man
a) ein Pyridylsulfonamid der Formel III 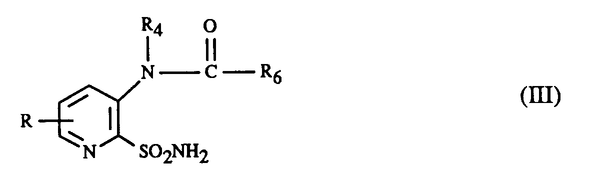 worin R, R₄ und R₆ die unter Formel I angegebene Bedeutung haben, mit einem N-Pyrimidinylcarbamat der Formel IV 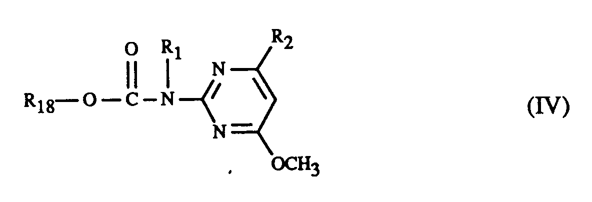 worin R₁ und R₂ die unter Formel I angegebene Bedeutung haben und R₁₈ C₁-C₄-Alkyl, oder Phenyl, welches durch C₁-C₄-Alkyl oder Halogen substituiert sein kann, bedeutet; in Gegenwart einer Base umsetzt; oder
b) ein 2-Pyridylsulfonylchlorid der Formel VII 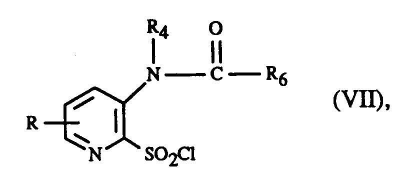 worin R, R₄ und R₆ die unter Formel I angegebene Bedeutung haben, mit einem Metall-Cyanat der Formel VIII

   O=C=N^{⊖}M^{⊕} (VIII),

   worin M^{⊕} ein Ammonium-, Phosphonium-, Sulfonium- oder ein Alkalimetall-Kation ist, und einem 2-Aminopyrimidin der Formel IX 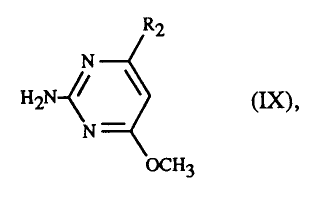 worin R₁ und R₂ die unter Formel I angegebene Bedeutung haben, in Gegenwart einer Base in einem inerten, organischen Lösungsmittel umsetzt, oder
c) ein Pyridylsulfonamid der Formel III 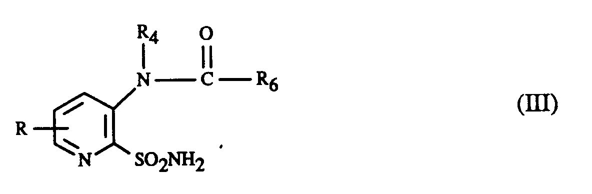 worin R, R₄ und R₆ die unter Formel I angegebene Bedeutung haben, in Gegenwart einer Base mit einem 2-Pyrimidinylisocyanat der Formel X 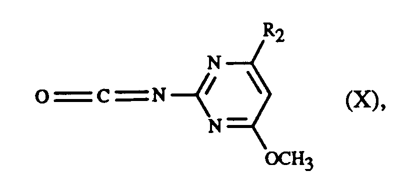 worin R₂ die unter Formel I angegebene Bedeutung hat, umsetzt.

Die Umsetzungen zu Verbindungen der Formel Ib werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Dichlormethan, Trichlormethan, Tetrachlormethan oder Chlorbenzol, Ether wie Diethylether, Ethylenglykoldimethylether, Diethylenglykoldimethylether, Tetrahydrofuran oder Dioxan, Nitrile wie Acetonitril oder Propionitril, Amide wie Dimethylformamid, Diethylformamid oder N-Methylpyrrolidinon. Die Reaktionstemperaturen liegen vorzugsweise zwischen -20° und +120°C.

Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triethylamin, Chinuclidin, 1,4-Diazabicyclo-(2.2.2)-octan, 1,5-Diazabicyclo(4.3.0)non-5-en oder 1,5-Diazabicyclo(5.4.0)undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte der Formel Ib können durch Einengen und/oder Verdampfen des Lösungsmittels isoliert und durch Umkristallisieren oder Zerreiben des festen Rückstandes in Lösungsmitteln, in denen sie sich nicht gut lösen, wie Ether, aromatischen Kohlenwasserstoffen oder chlorierten Kohlenwasserstoffen, gereinigt werden.

Die 2-Pyrimidinylisocyanate der Formel X können aus den entsprechenden 2-Aminopyrimidinen nach bekannten Methoden hergestellt werden. Derartige Verfahren sind beispielsweise in US-A-3,919,228 sowie in US-A-3,732,223 beschrieben.

Die Zwischenprodukte der Formel IV können analog zu bekannten Verfahren hergestellt werden. Die Verbindungen der Formel III sind bekannt oder nach bekannten Methoden herstellbar. Verbindungen der Formel III sind z.B. aus EP-A-0 555 770 bekannt und können auch analog den in der EP-A-0 314 505, EP-A-0 459 949 beschriebenen Verfahren hergestellt werden. Verfahren zur Herstellung von N-Pyrimidinylcarbamaten sind beispielsweise in EP-A-0 101 670 beschrieben.

Verbindungen der Formel III lassen sich beispielsweise herstellen, indem man eine Verbindung der Formel V 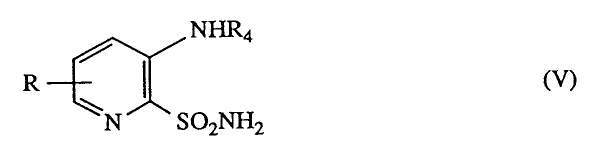 worin R und R₄ die unter Formel I angegebene Bedeutung haben, mit einem Acylierungsmittel der Formel VI

Z-R₅ (VI)

worin R₅ die Gruppe C(O)-R₆ bedeutet, R₆ die unter Formel I angegebene Bedeutung hat und Z eine Abgangsgruppe wie z.B. Halogen, R₅O-, R₆C(O)O- oder Imidazol bedeutet, in Gegenwart einer Base umsetzt. Derartige Umsetzungen sind beispielsweise in Farmaco Ed. scient. 12, 392 (1957) beschrieben. Die Verbindungen der Formel V sind entweder bekannt oder können nach bekannten Methoden, die beispielsweise in der EP-A-0 459 949 offenbart sind, hergestellt werden.

Pyridylsulfonamide der Formel III können auch aus den entsprechenden 2-Pyridylsulfonylchloriden der Formel VII durch Umsetzung mit Ammoniak hergestellt werden.

2-Pyridylsulfonylchloride der Formel VII können ganz besonders vorteilhaft hergestellt werden, indem man eine Verbindung der Formel XI 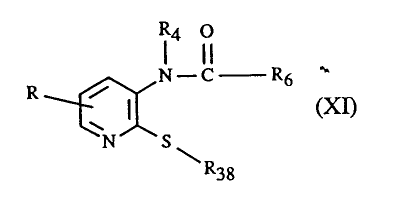 worin R, R₄ und R₆ die unter Formel I angegebenen Bedeutungen haben und R₃₈ Benzyl, Methoxymethyl oder Isopropyl bedeutet, mit wäßrigem Chlor umsetzt.

Die speziell für die vorliegende Erfindung entwickelten Zwischenprodukte der Formel XI erlauben eine ökonomisch besonders vorteilhafte Herstellung der Verbindungen der Formel VII bzw. III, die zur Herstellung des Herbizids der Formel Ib, das in bevorzugten erfindungsgemäßen Mitteln enthalten ist, benötigt werden.

Die Verbindungen der Formel XI werden hergestellt, indem man eine Verbindung der Formel XII 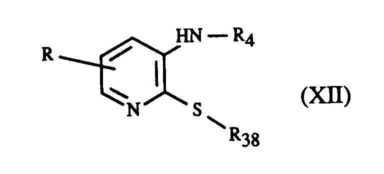 worin R, R₄ und R₃₈ die oben angegebenen Bedeutungen haben, mit einer Verbindung der Formel VI

Z-R₅ (VI)

worin R₅ die Gruppe C(O)-R₆ bedeutet, R₆ die unter Formel I angegebene Bedeutung hat und Z eine Abgangsgruppe wie z.B. Halogen, R₅O-, R₆C(O)O- oder Imidazol bedeutet, bedeutet, in Gegenwart einer Base umsetzt.

Verbindungen der Formel XII können hergestellt werden, indem man z B. eine Verbindung der Formel XIV 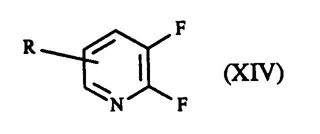 worin R die unter Formel I angegebene Bedeutung hat, mit Benzyl- oder Isopropylmercaptan in Gegenwart einer Base zu einer Verbindung der Formel XIII 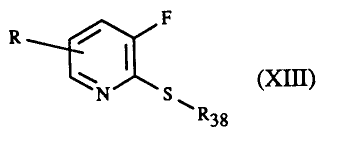 worin R₃₈ Benzyl, Methoxymethyl oder Isopropyl bedeutet und R die unter Formel I angegebene Bedeutung hat, umsetzt, und diese in Gegenwart einer Base durch Reaktion mit der Verbindung der Formel XV

H₂NR₄ (XV),

worin R₄ die unter Formel I angegebene Bedeutung hat, in die Verbindung der Formel XII überführt.

Die Erfindung betrifft auch ein Verfahren zum selektiven Bekämpfen von Unkräutern in Nutzpflanzenkulturen, welches darin besteht, daß man die Nutzpflanzen bzw. deren Anbaufläche gleichzeitig oder unabhängig voneinander mit einer herbizid wirksamen Menge der Pyridylsulfonylharnstoffe der Formel I und einer herbizid-antagonistisch wirksamen Menge eines Sulfamoylphenylharnstoffes der Formel II behandelt.

Als Kulturpflanzen, welche gegen die schädigende Wirkung der oben erwähnten Herbizide der Formel I geschützt werden können, kommen insbesondere diejenigen in Betracht, die auf dem Nahrungs- oder Textilsektor von Bedeutung sind, beispielsweise Zuckerrohr und insbesondere Kulturhirse und Mais, sowie Reis und andere Getreidearten wie Weizen, Roggen, Gerste und Hafer. Das erfindungsgemäße Mittel eignet sich besonders gut zum Schützen von Maiskulturen und Zuckerrohrkulturen vor der herbiziden Wirkung der Verbindungen der Formel I.

Bei den zu bekämpfenden Unkräutern kann es sich sowohl um monokotyle wie um dikotyle Unkräuter handeln.

Als Kulturpflanzen oder Teile dieser Pflanzen kommen beispielsweise die vorstehend genannten in Betracht. Als Anbauflächen gelten die bereits mit den Kulturpflanzen bewachsenen oder mit dem Saatgut dieser Kulturpflanzen beschickten Bodenareale, wie auch die zur Bebauung mit diesen Kulturpflanzen bestimmten Böden.

Ein Safener der Formel II kann je nach Anwendungszweck zur Vorbehandlung des Saatgutes der Kulturpflanze (Beizung des Samens oder der Stecklinge) eingesetzt oder vor oder nach der Saat in den Boden gegeben werden. Er kann aber auch für sich allein oder zusammen mit dem Herbizid nach dem Auflaufen der Pflanzen appliziert werden. Die Behandlung der Pflanzen oder des Saatgutes mit dem Safener kann daher grundsätzlich unabhängig vom Zeitpunkt der Applikation des Herbizids erfolgen. Die Behandlung der Pflanze kann man jedoch auch durch gleichzeitige Applikation von Herbizid und Safener (z.B. als Tankmischung) vornehmen.

Die zu applizierende Aufwandmenge Safener im Verhältnis zum Herbizid richtet sich weitgehend nach der Anwendungsart. Bei einer Feldbehandlung, welche entweder unter Verwendung einer Tankmischung mit einer Kombination von Safener und Herbizid oder durch getrennte Applikation von Safener und Herbizid erfolgt, liegt in der Regel ein Verhältnis von Safener zu Herbizid von 1:100 bis 1:1, bevorzugt 1:20 bis 1:1, und insbesondere 1:1, vor. Dagegen werden bei der Samenbeizung weit geringere Mengen Safener im Verhältnis zur Aufwandmenge an Herbizid pro Hektar Anbaufläche benötigt.

In der Regel werden bei der Feldbehandlung 0,001 bis 5,0 kg Safener/ha, vorzugsweise 0,005 bis 0,5 kg Safener/ha, appliziert.

Die Aufwandmengen an Herbizid liegt in der Regel zwischen 0,001 bis 2 kg/ha, vorzugsweise jedoch zwischen 0,001 bis 0,5 kg/ha.

Bei der Samenbeizung werden im allgemeinen 0,001 bis 10 g Safener/kg Samen, vorzugsweise 0,05 bis 2 g Safener/kg Samen, appliziert. Wird der Safener in flüssiger Form kurz vor der Aussaat unter Samenquellung appliziert, so werden zweckmäßigerweise Safener-Lösungen verwendet, welche den Wirkstoff in einer Konzentration von 1 bis 10000, vorzugsweise von 100 bis 1000 ppm, enthalten.

Zur Applikation werden die Verbindungen der Formel II oder Kombinationen von Verbindungen der Formel II mit den zu antagonisierenden Herbiziden zweckmäßigerweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen, Bestreichen oder Gießen werden gleich wie die Art der zu verwendenden Mittel den angestrebten Zielen und den gegebenen Verhältnissen entsprechend gewählt.

Die Formulierungen, d.h. die den Wirkstoff der Formel II oder eine Kombination von Wirkstoff der Formel II mit zu antagonisierendem Herbizid der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen C₈ bis C₁₂, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole, sowie deren Ether und Ester wie Ethanol, Ethylenglykol, Ethylenglykolmonomethyl- oder ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnußöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffs der Formel II und gegebenenfalls auch dem zu antagonisierenden Herbizid der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren (C₁₀-C₂₂), wie z.B. die Na- oder K-Salze der Oel- oder Stearinsäure, oder von natürlichen Fettsäuregemischen, die z.B. aus Kokosnuß-oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettallrohol-Ethyltnoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, , Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte and Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylen-sorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, , Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood New Jersey, 1981. Stache, H., "Tensid-Taschenbuch", Carl Hanser Verlag, München/Wien, 1981.

Die agrochemischen Zubereitungen enthalten in der Regel 0,1 bis 99 Gewichtsprozent, insbesonder 0,1 bis 95 Gew.-%, Wirkstoff der Formel II oder Wirkstoffgemisch Antidot/Herbizid, 1 bis 99,9 Gew.-%, insbesonder 5 bis 99,8 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Für die Verwendung von Verbindungen der Formel II oder sie enthaltender Mittel zum Schützen von Kulturpflanzen gegen schädigende Wirkungen von Herbiziden der Formel I kommen verschiedene Methoden und Techniken in Betracht, wie beispielsweise die folgenden:

### i) Samenbeizung

a) Beizung der Samen mit einem als Spritzpulver formulierten Wirkstoff der Formel II durch Schütteln in einem Gefäß bis zur gleichmäßigen Verteilung auf der Samenoberfläche (Trockenbeizung). Man verwendet dabei etwa 1 bis 500 g Wirkstoff der Formel II (4 g bis 2 kg Spritzpulver) pro 100 kg Saatgut.
b) Beizung der Samen mit einem Emulsionskonzentrat des Wirkstoffs der Formel II nach der Methode a) (Naßbeizung).
c) Beizung durch Tauchen des Saatguts in eine Brühe mit 100-1000 ppm Wirkstoff der Formel II während 1 bis 72 Stunden und gegebenenfalls nachfolgendes Trocknen der Samen (Tauchbeizung).

Die Beizung des Saatguts oder die Behandlung des angekeimten Sämlings sind naturgemäß die bevorzugten Methoden der Applikation, weil die Wirkstoffbehandlung vollständig auf die Zielkultur gerichtet ist. Man verwendet in der Regel 1 bis 1000 g Antidot, vorzugsweise 5 bis 250 g Antidot, pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

### ii) Applikation aus Tankmischung

Eine flüssige Aufarbeitung eines Gemisches von Antidot und Herbizid (gegenseitiges Mengenverhältnis zwischen 10:1 und 1:100) wird verwendet, wobei die Aufwandmenge an Herbizid 0,01 bis 5,0 kg pro Hektar beträgt. Solche Tankmischung wird vor oder nach der Aussaat appliziert.

### iii) Applikation in der Saatfurche

Das Antidot wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

### iv) Kontrollierte Wirkstoffabgabe

Der Wirkstoff der Formel II wird in Lösung auf mineralische Granulatträger oder polymerisierte Granulate (Harnstoff/Formaldehyd) aufgezogen und getrocknet.

Gegebenenfalls kann ein Überzug aufgebracht werden (Umhüllungsgranulate), der es erlaubt, den Wirkstoff über einen bestimmten Zeitraum dosiert abzugeben.

Die Verbindungen der Formeln I und II können sowohl getrennt als auch gemeinsam formuliert werden. Insbesondere setzen sich bevorzugte Formulierungen folgendermaßen zusammen:
(% = Gewichtsprozent)

### Emulgierbare Konzentrate:

- Aktiver Wirkstoff:: 1 bis 20 %, vorzugsweise 5 bis 10 %
- oberflächenaktives Mittel:: 5 bis 30 %, vorzugsweise 10 bis 20 %
- flüssiges Trägermittel:: 15 bis 94 %, vorzugsweise 70 bis 85 %

### Stäube:

- Aktiver Wirkstoff:: 0,1 bis 10 %, vorzugsweise 0,1 bis 1 %
- festes Trägermittel:: 99,9 bis 90 %, vorzugsweise 99,9 bis 99 %

### Suspensions-Konzentrate:

- Aktiver Wirkstoff:: 5 bis 75 %, vorzugsweise 10 bis 50 %
- Wasser:: 94 bis 24 %, vorzugsweise 88 bis 30 %
- oberflächenaktives Mittel:: 1 bis 40 %, vorzugsweise 2 bis 30 %

### Benetzbare Pulver:

- Aktiver Wirkstoff:: 0,5 bis 90 %, vorzugsweise 1 bis 80 %
- oberflächenaktives Mittel:: 0,5 bis 20 %, vorzugsweise 1 bis 15 %
- festes Trägermaterial:: 5 bis 95 %, vorzugsweise 15 bis 90 %

### Granulate:

- Aktiver Wirkstoff:: 0,5 bis 30 %, vorzugsweise 3 bis 15 %
- festes Trägermittel:: 99,5 bis 70 %, vorzugsweise 97 bis 85 %

### Herstellungsbeispiele:

### Beispiel H1: Herstellung von 3-(N-Methyl-N-methylcarbonylamino)-pyridin-2-yl-sulfonamid:

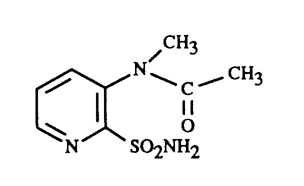

Zu einer Lösung von 1,87g 3-N-Methylaminopyridin-2-yl-sulfonamid in 40 ml trockenem Acetonitril gibt man bei Raumtemperatur nacheinander 0,89 ml Pyridin und 0,82 ml Acetylbromid hinzu. Nach 30 Minuten gibt man weitere 0,3 ml Acetylbromid hinzu. Nach 60-minütigem Rühren gibt man 0,32 ml Pyridin hinzu. Nach 30-minütigem Rühren wird die Reaktionsmischung filtriert. Man erhält 0,8 g 3-(N-Methyl-N-methylcarbonylamino)-pyridin-2-yl-sulfonamid mit einem Schmelzpunkt von 181 bis 185°C.

### Beispiel H2: Herstellung von N-(N-Methyl-N-methylcarbonylamino)-pyridin-2-yl-sulfonyl-N'-(4-methoxy-6-methylpyrimidin-2-yl)harnstoff(Verbindung Nr. 1.01):

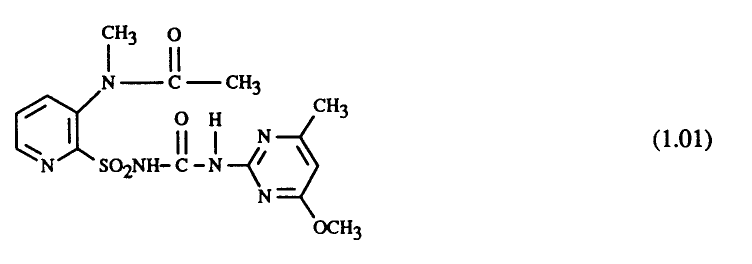

Zu einer Lösung von 3,07 g 3-(N-Methyl-N-methylcarbonylamino)-pyridin-2-yl-sulfonamid in 50 ml Acetonitril gibt man nacheinander 2,18 ml 1,5-Diazabicyclo[5.4.0]undec-5-en und 3,89 g N-(4-Methoxy-6-methylpyrimidin-2-yl)-phenylcarbamat hinzu. Nach 45-minütigem Rühren wird die Reaktionsmischung im Vakuum eingeengt, der ölige Rückstand mit 10 ml 2N Salzsäure verrieben und anschließend mit 10 ml Wasser verdünnt. Das kristalline Produkt wird filtriert und anschließend mit Wasser und Diethylether gewaschen. Man erhält 5,25 g N-(N-Methyl-N-methylcarbonylamino)-pyridin-2-yl-sulfonyl-N'-(4-methoxy-6-methylpyrimidin-2-yl)harnstoff (Verbindung Nr. 1.01) mit einem Schmelzpunkt von 178 bis 180°C.

### Beispiel H3: Herstellung von 3-(N-Methyl-N-methylcarbonylamino)-pyridin-2-yl-sulfonylchlorid:

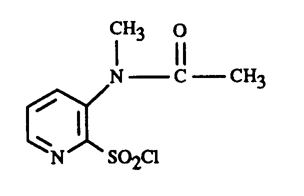

In eine Emulsion von 3,6g 3-(N-Methyl-N-methylcarbonylamino)-2-isopropylthiopyridin in 35 ml Methylenchlorid und 48 ml 1N Salzsäure leitet man bei einer Temperatur von - 5 °C innerhalb von 15 Minuten 4,6g Chlorgas ein und rührt für weitere 15 Minuten. Anschließend leitet man innerhalb von 20 Minuten bei gleicher Temperatur Stickstoff durch die Reaktionsmischung. Nach Trennen der Phasen und dreimaligem Waschen mit Methylenchlorid und Eiswasser wird die Reaktionsmischung mit Natriumsulfat getrocknet und filtriert. Die so erhaltene Lösung von N-(N-Methyl-N-methylcarbonylamino)-pyridin-2-yl-sulfonylchlorid (60 ml) kann entweder direkt zum entsprechenden Sulfonamid (Beispiel H1) oder zu den Sulfonylharnstoffen der Formel I, worin R für Waserstoff, und R₄ und R₆ jeweils für Methyl stehen, umgesetzt werden.

### Beispiel H4: Herstellung von 3-(N-Methyl-N-methylcarbonylamino)-2-isopropylthiopyridin:

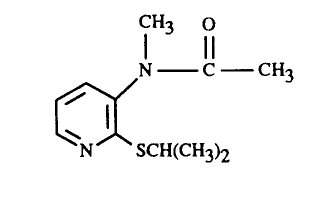

Zu einer Lösung von 2,73g 3-N-Methyl-2-isopropylthiopyndin in 40 ml Acetonitril gibt man bei einer Temperatur von 0 bis 2°C 1,34 ml Acetylbromid tropfenweise hinzu. Anschließend wird die Reaktionsmischung 20 Minuten lang bei 0°C gerührt. Nach einer Zugabe von 1,33 ml Pyridin und einstündigem Rühren wird die Reaktionsmischung eingeengt und mit Essigsäureethylester/Wasser versetzt. Nach 3-maligem Waschen mit Essigsäureethylester und einmaligem Waschen mit Wasser wird die Reaktionsmischung über Natriumsulfat getrocknet und anschließend eingedampft. Man erhält 3,25 g N-(N-Methyl-N-methylcarbonylamino)-2-isopropylthiopyridin, welches nach Umkristallisation aus Petrolether einen Schmelzpunkt von 49 bis 51°C aufweist.

### Beispiel H5: Herstellung von 3-N-Methylamino-2-isopropylthiopyridin:

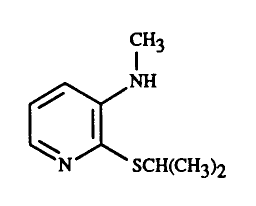

Zu 8,56g 3-Fluor-2-isopropylthiopyridin preßt man in einem Druckgefäß 45 g Methylamingas und hält die Reaktionsmischung 24 Stunden bei einer Temperatur von 140 °C und anschließend 16 Stunden bei 150 °C. Nach Filtrieren und Eindampfen des Reaktionsgemisches wird mittels Säulenchromatographie (Essigsäureethylester/Hexan 1: 9) gereinigt. Man erhält 6,77g 3-N-Methylamino-2-isopropylthiopyridin mit einem Schmelzpunkt von 37 bis 38 °C.

### Beispiel H6: Herstellung von 3-Fluor-2-isopropylthiopyridin:

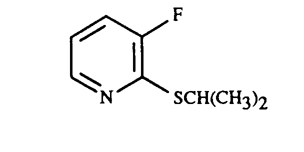

Zu einem Gemisch von 388,6 g Kaliumcarbonat und 301 g 2,3-Difluorpyridin in 4300 ml Dimethylformamid gibt man bei einer Temperatur von 0 bis -5°C 220,93 g Isopropylmercaptan tropfenweise hinzu. Nach 20-minütigem Rühren läßt man die Reaktionsmischung langsam auf Raumtemperatur erwärmen und rührt für weitere 30 Minuten. Anschließend gibt man die Reaktionsmischung in ein Eiswasser/Essigsäureethylestergemisch, wäscht die Phasen viermal mit Essigsäureethylester und Eiswasser und trocknet anschließend über Natriumsulfat. Nach Filtrieren und Eindampfen wird das so erhaltene Rohprodukt (510 g) säulenchromatographisch gereinigt (Essigsäureethylester/Hexan 1 : 9). Man erhält 327,3 g 3-Fluor-2-isopropylthiopyridin in Form eines Öls.

In analoger Weise werden die in den angeschlossenen Tabellen aufgelisteten Verbindungen der Formel I, Ib und III hergestellt.

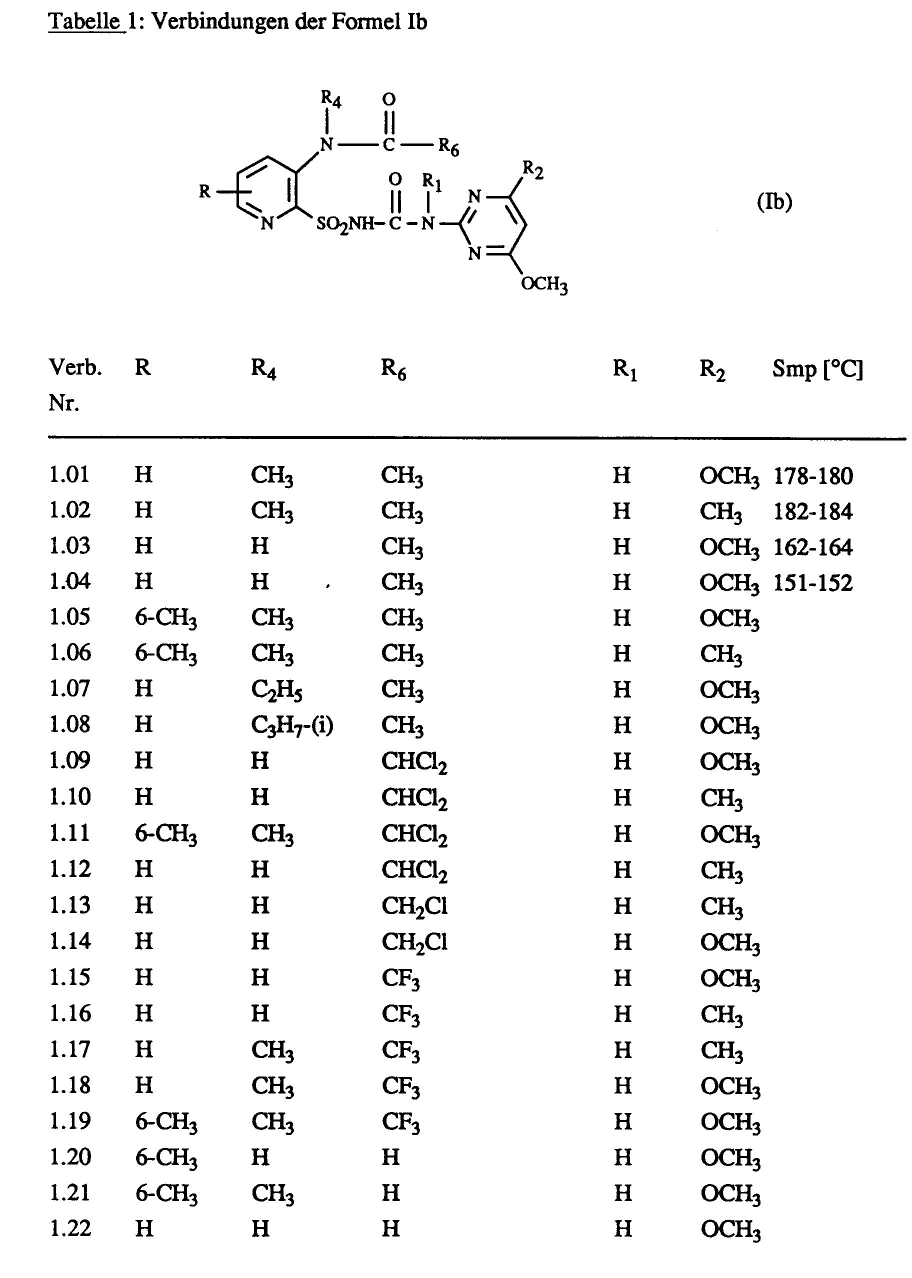

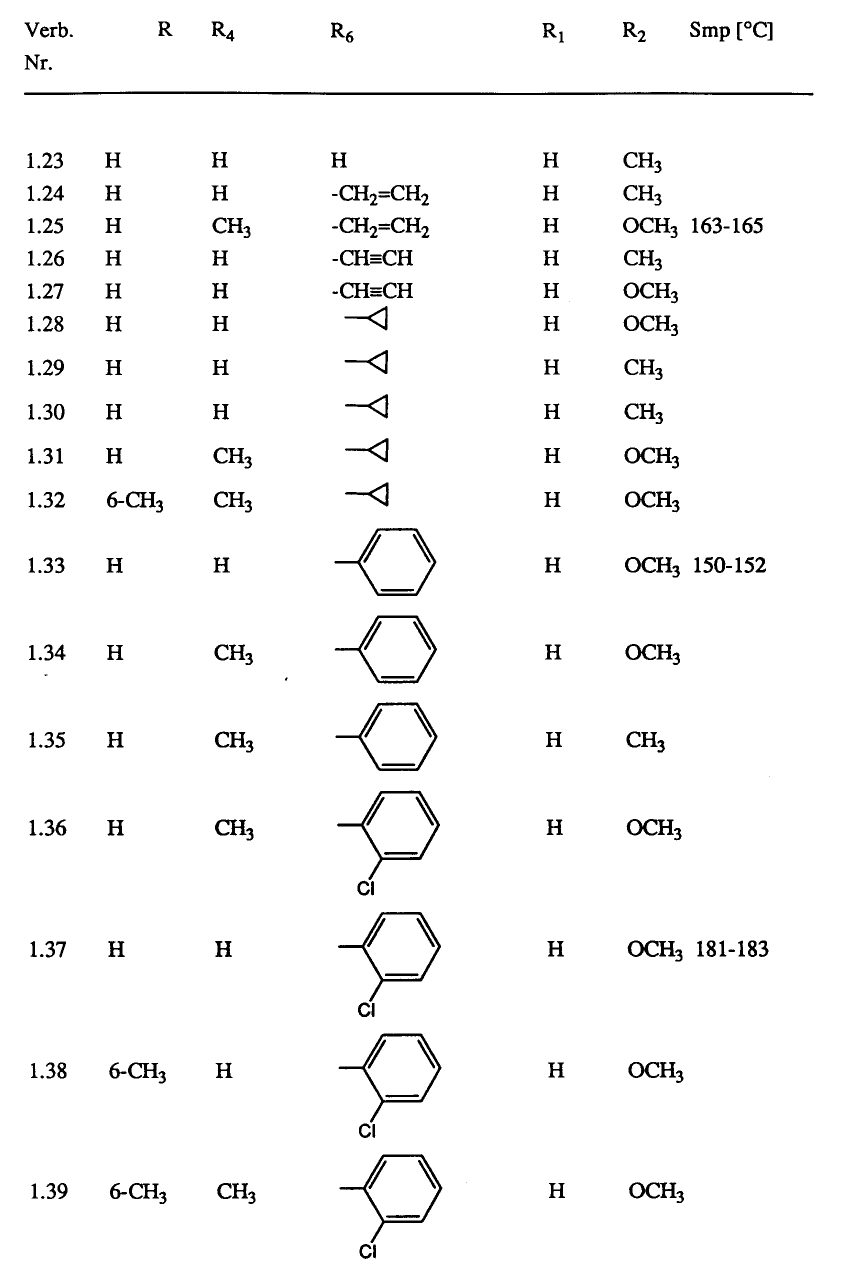

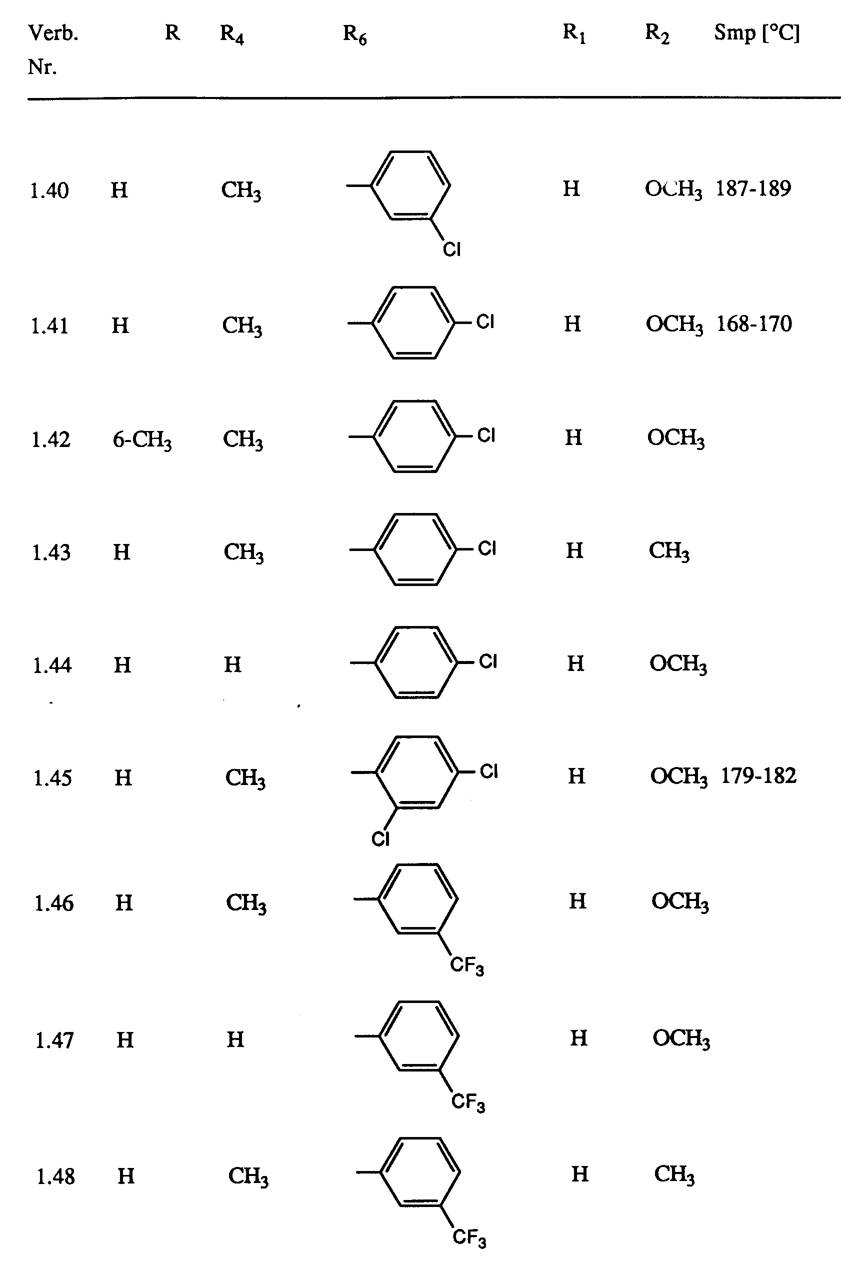

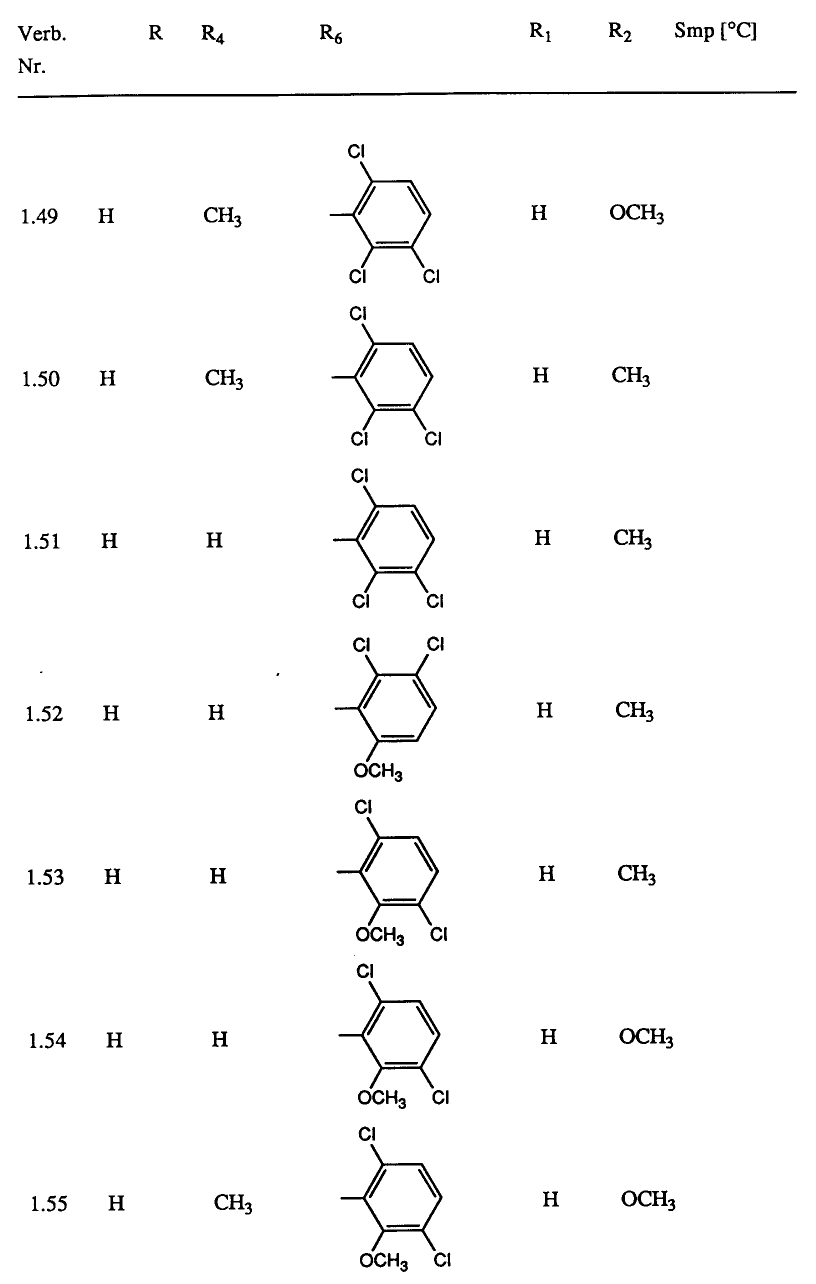

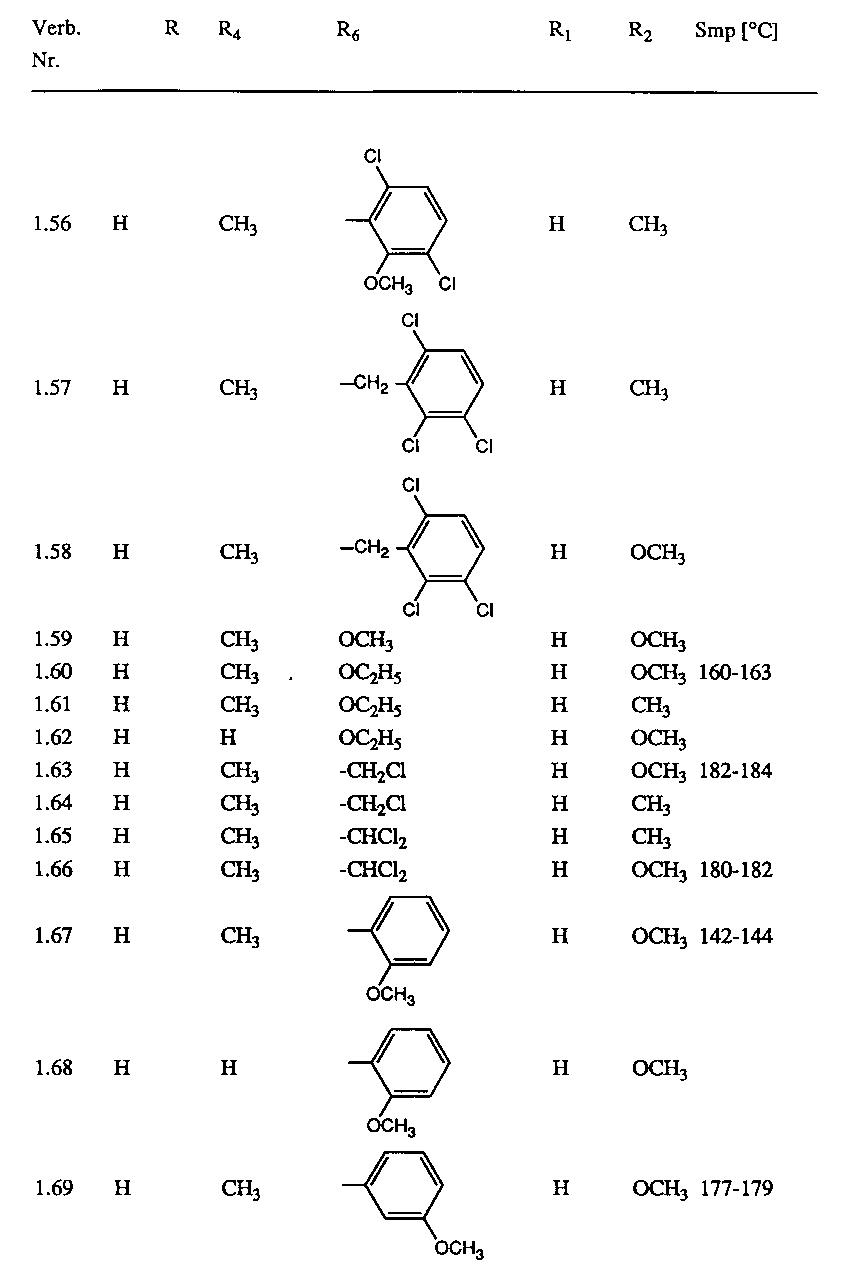

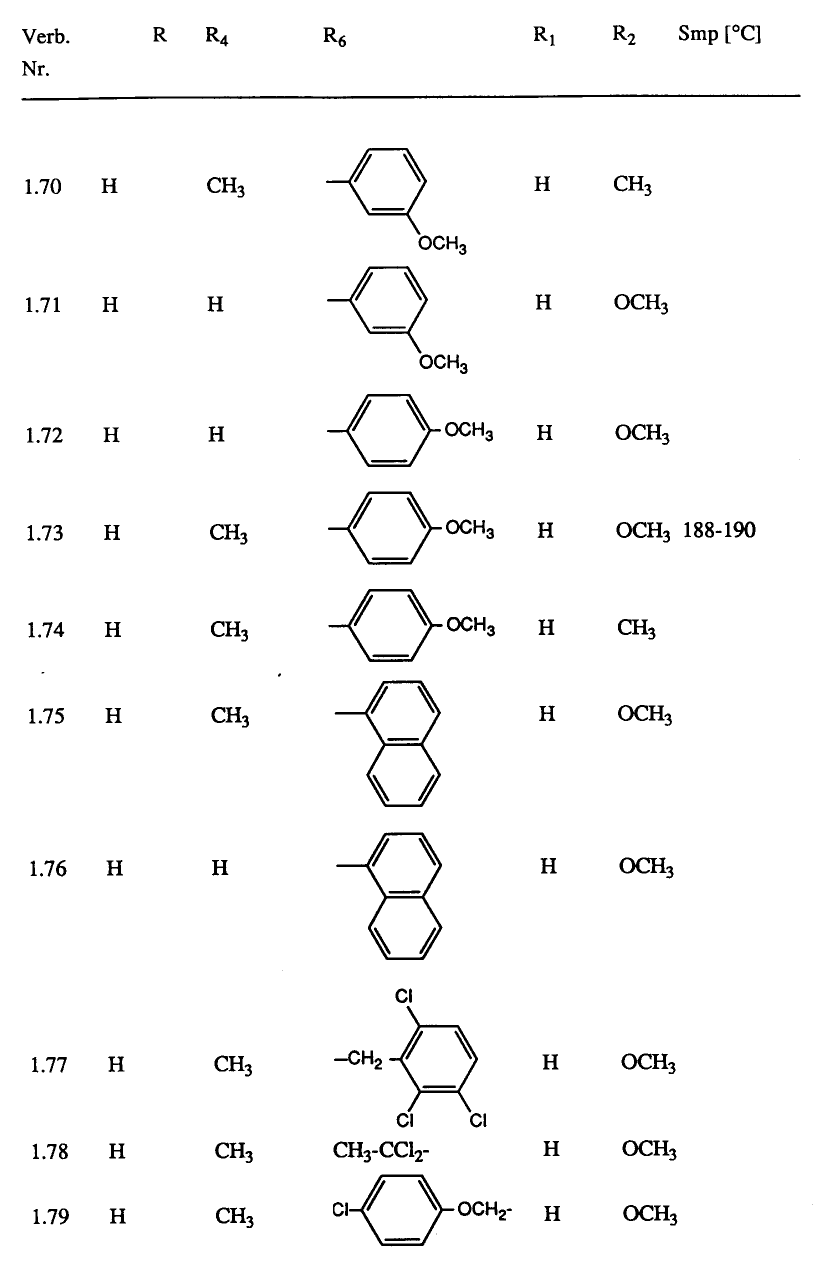

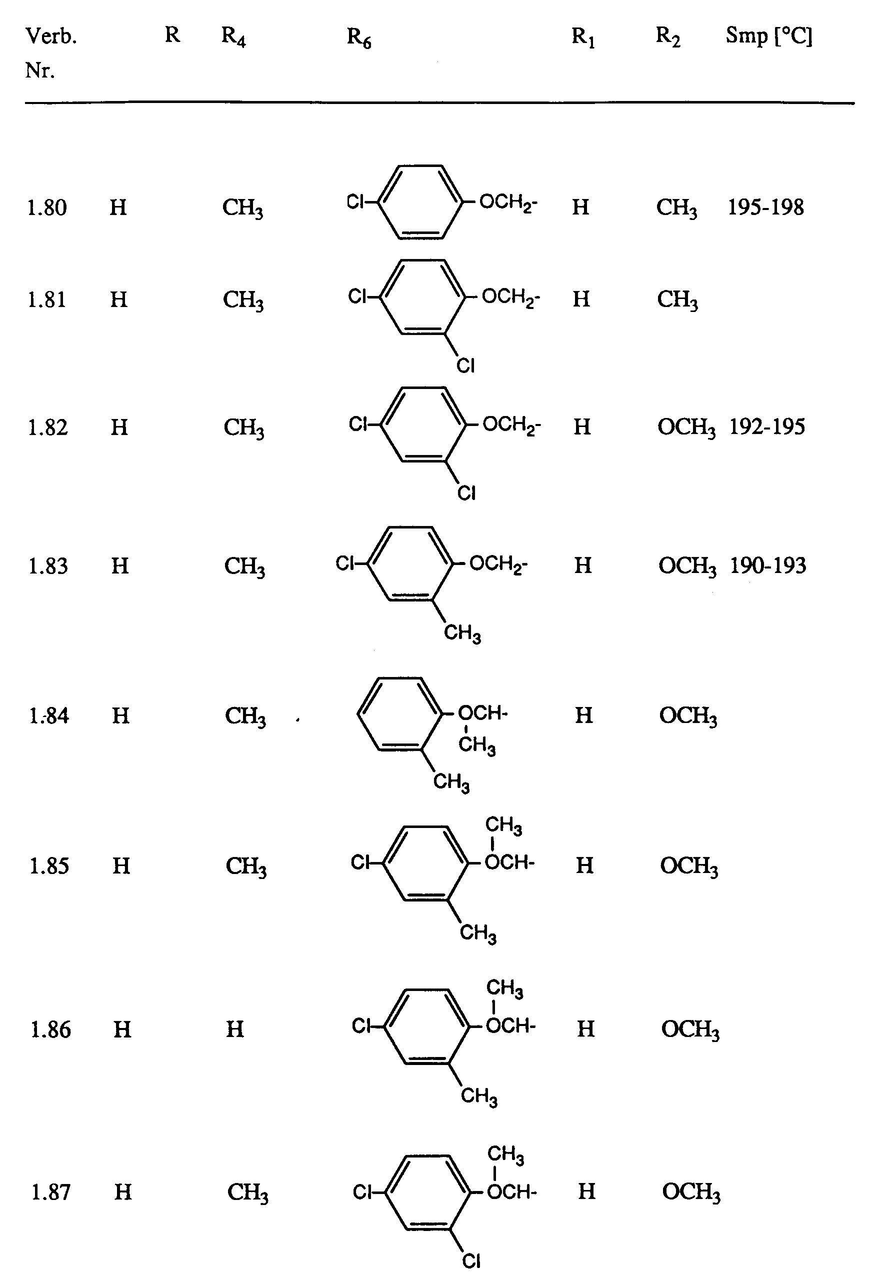

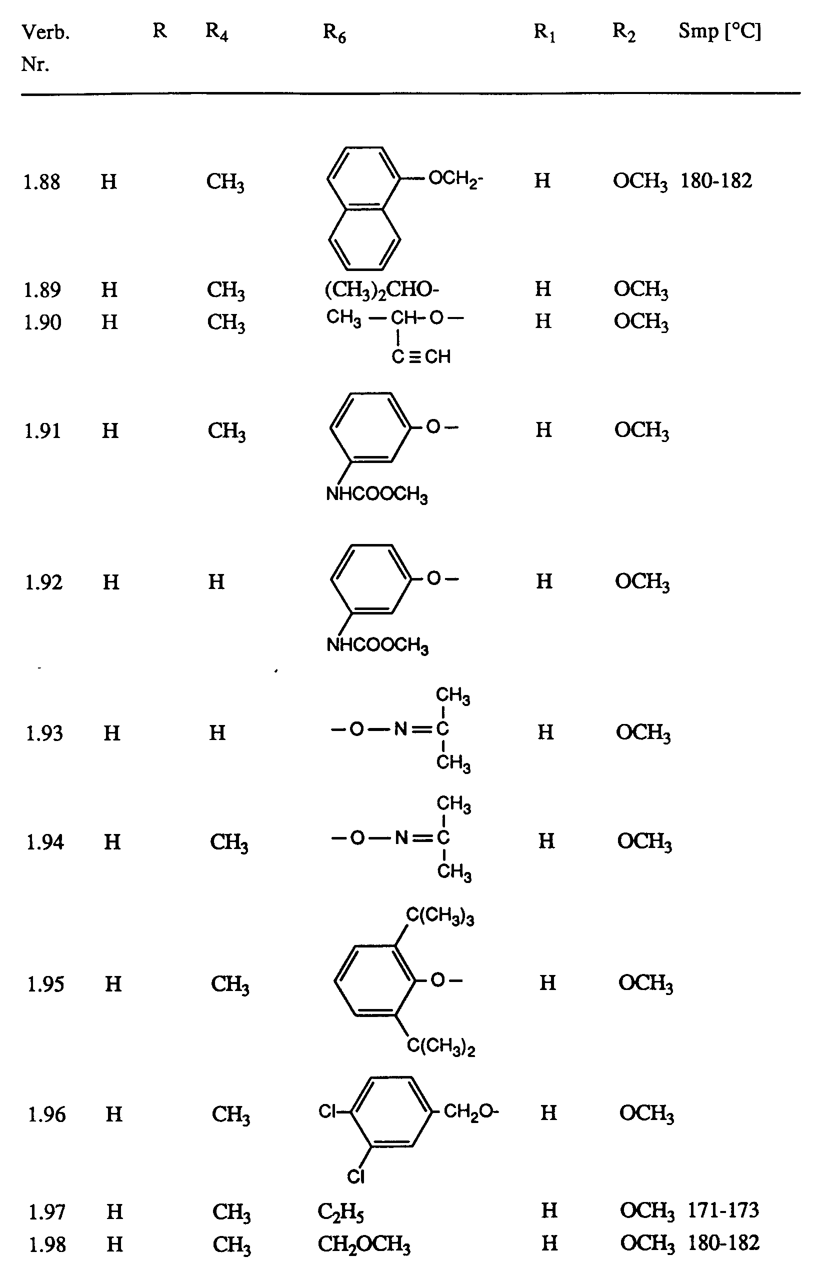

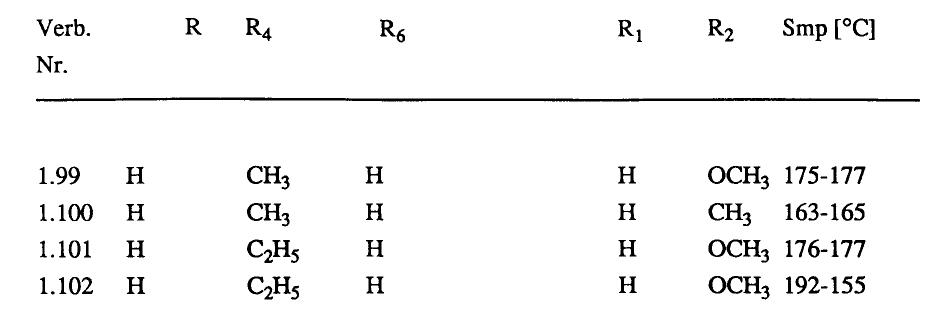

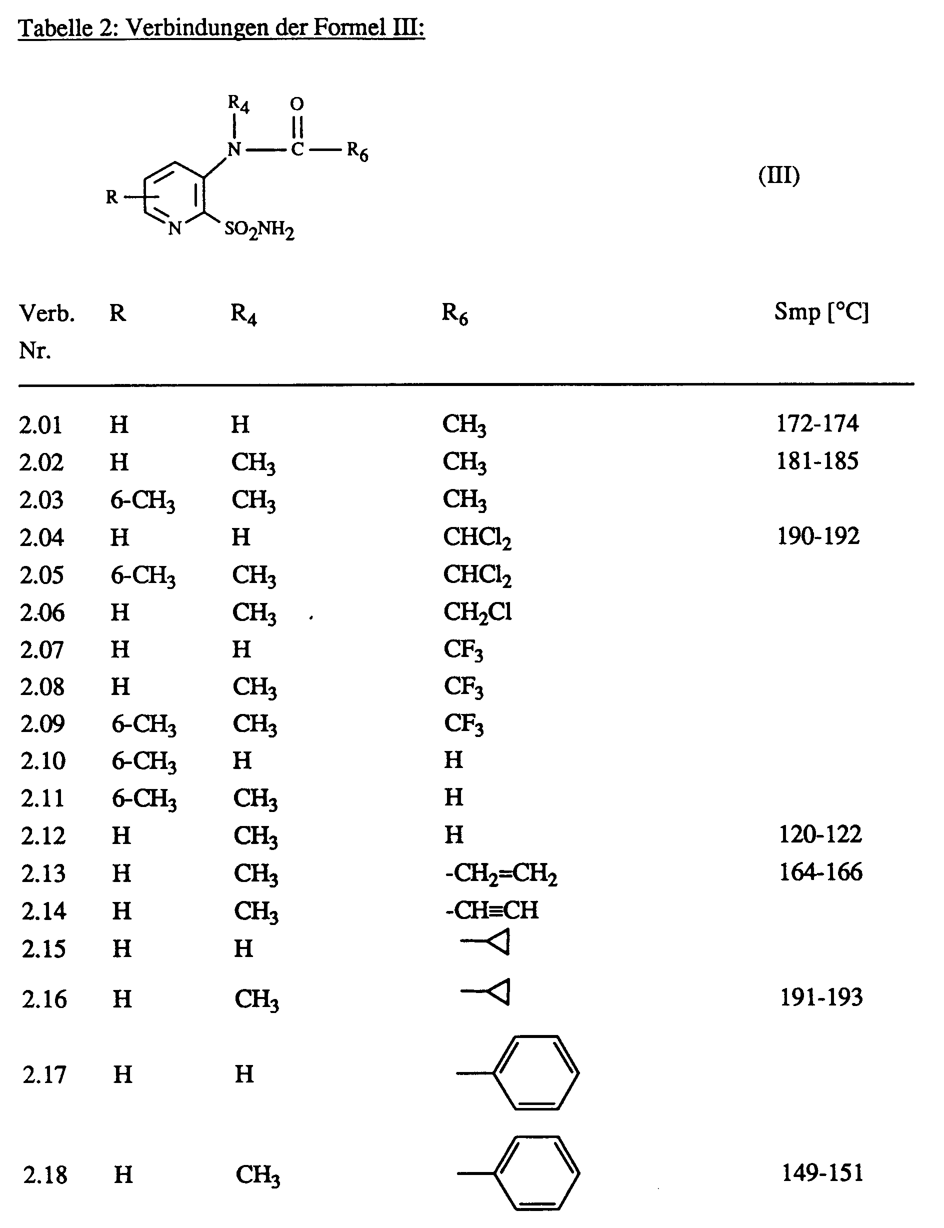

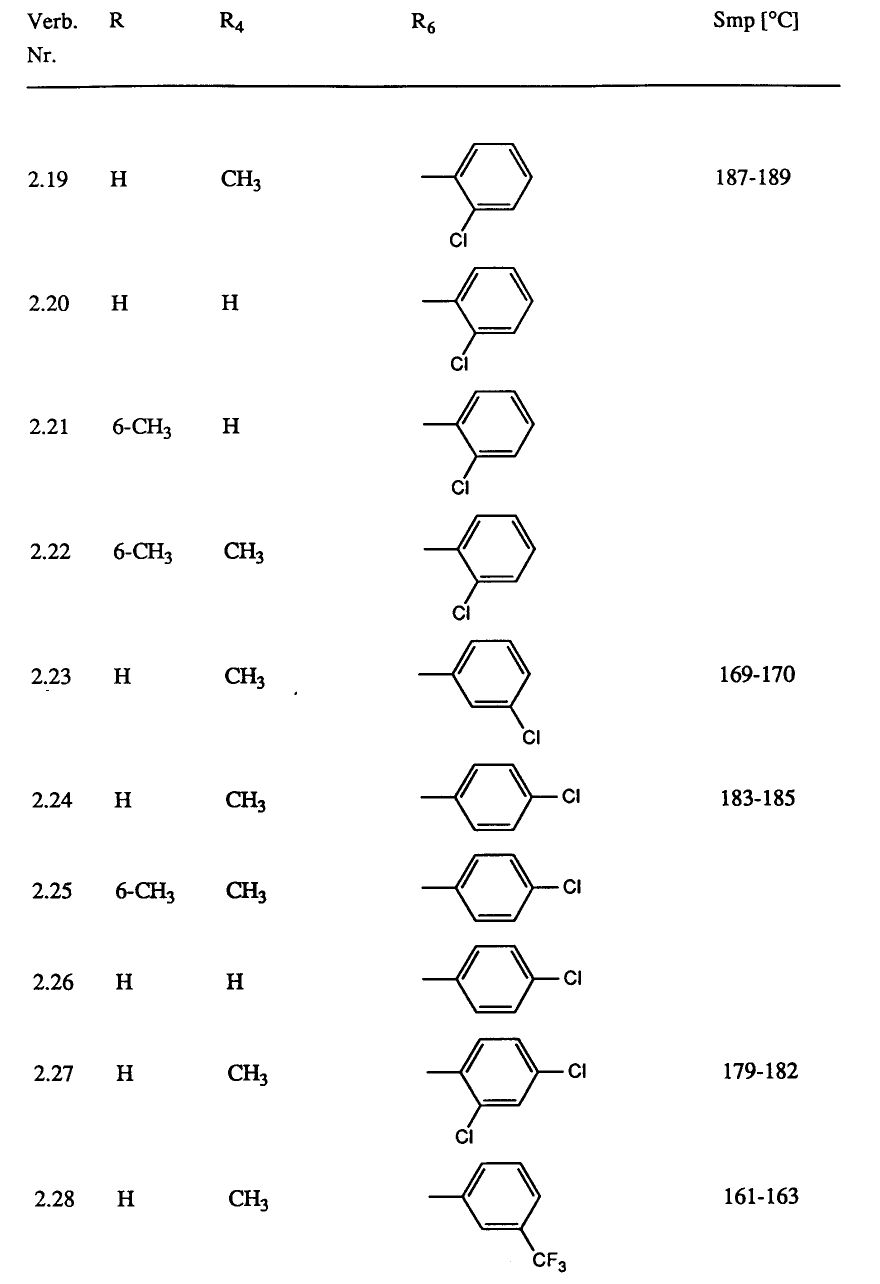

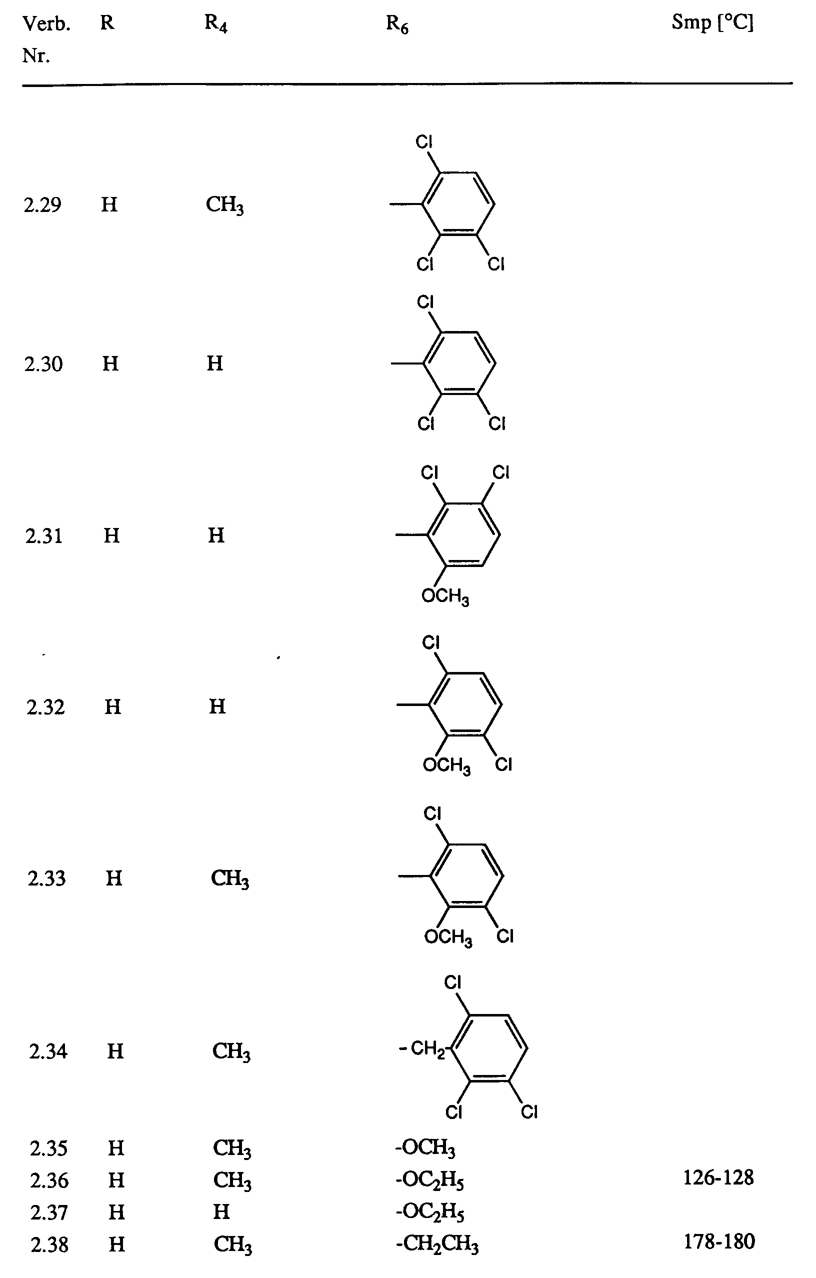

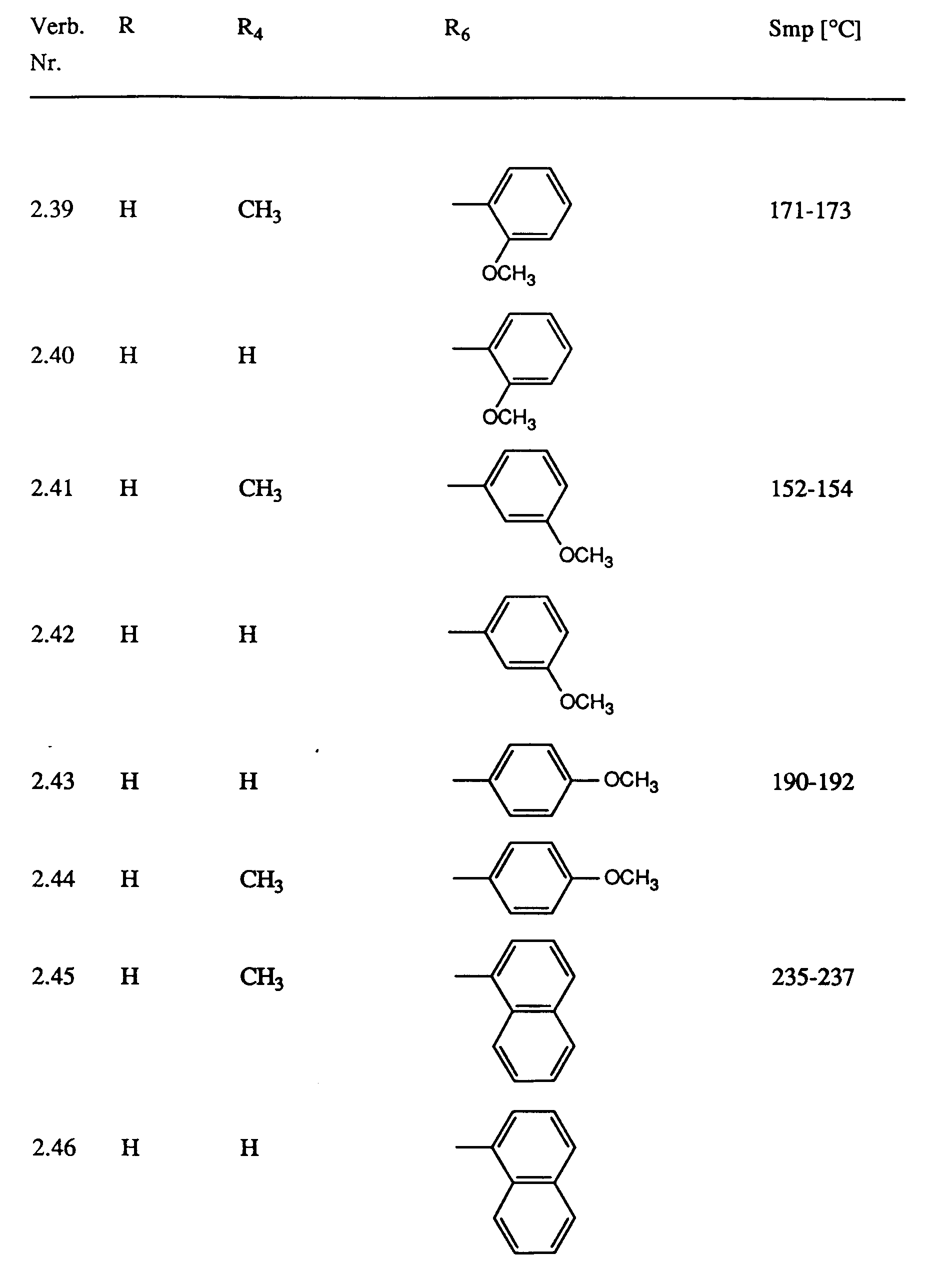

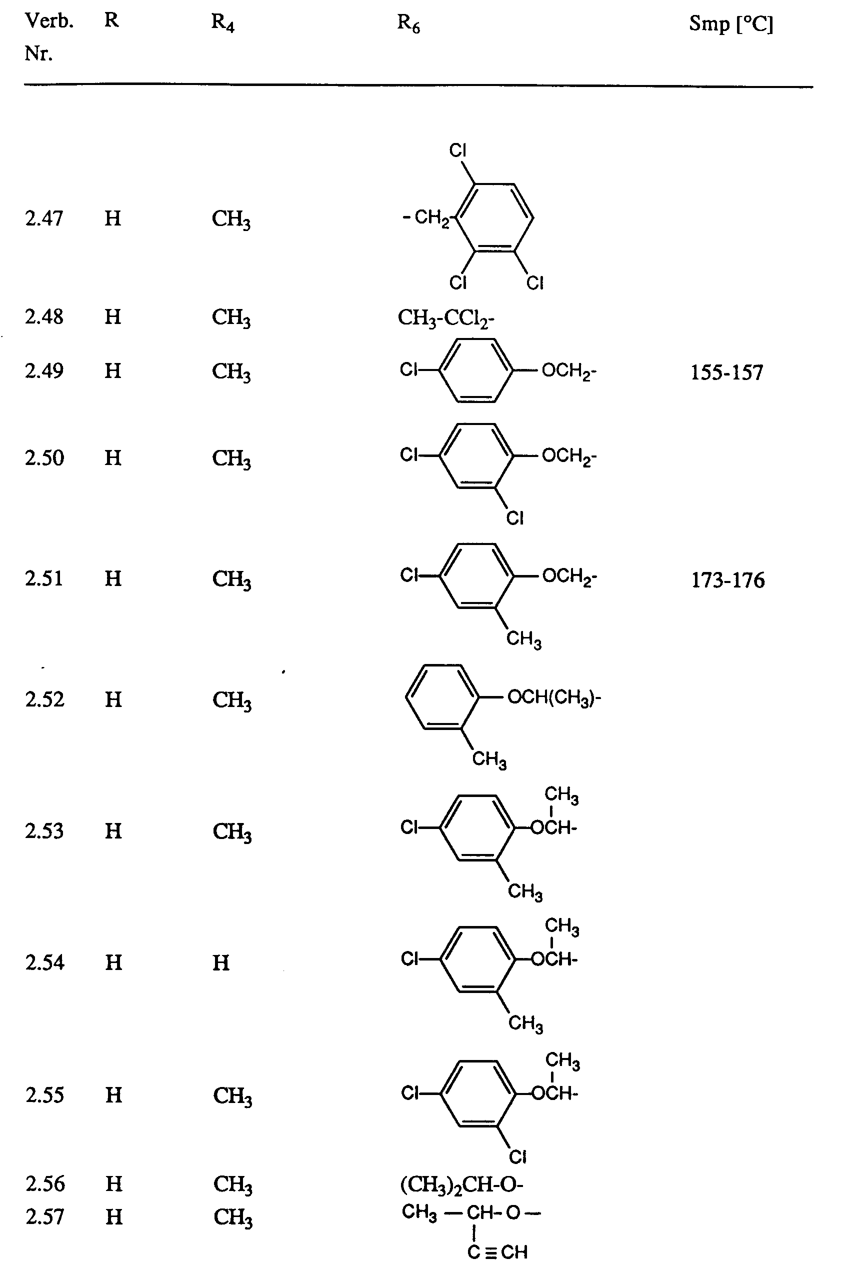

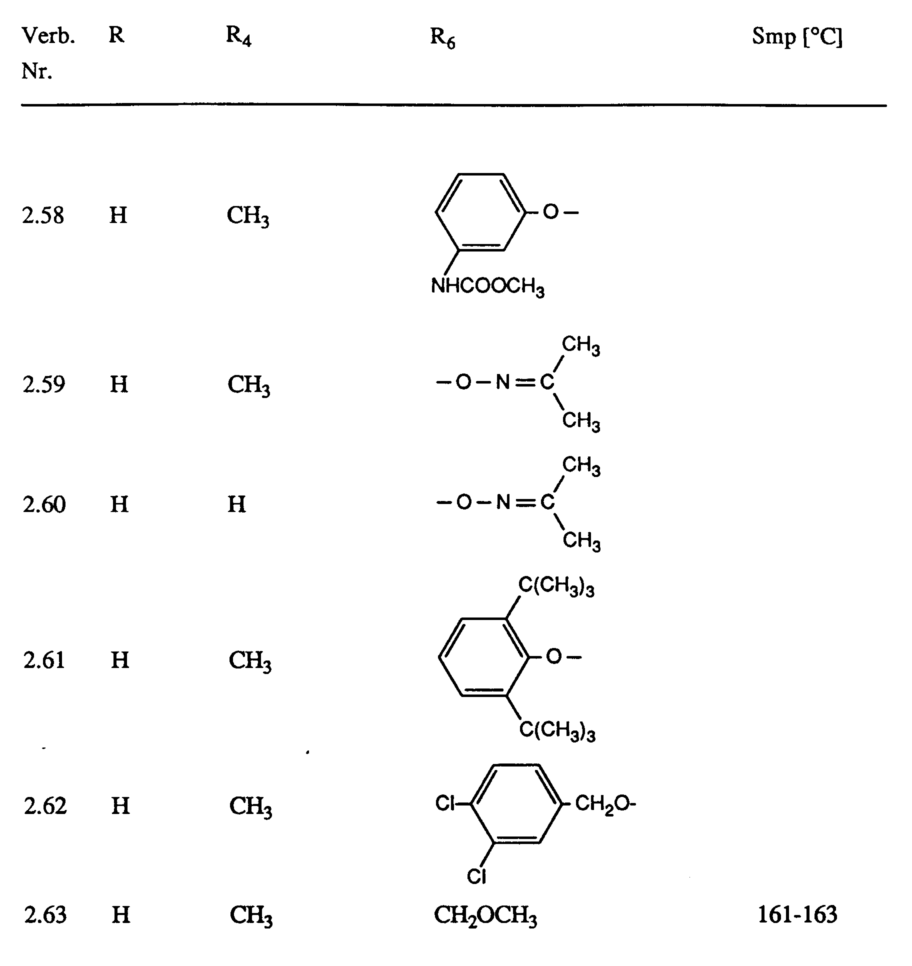

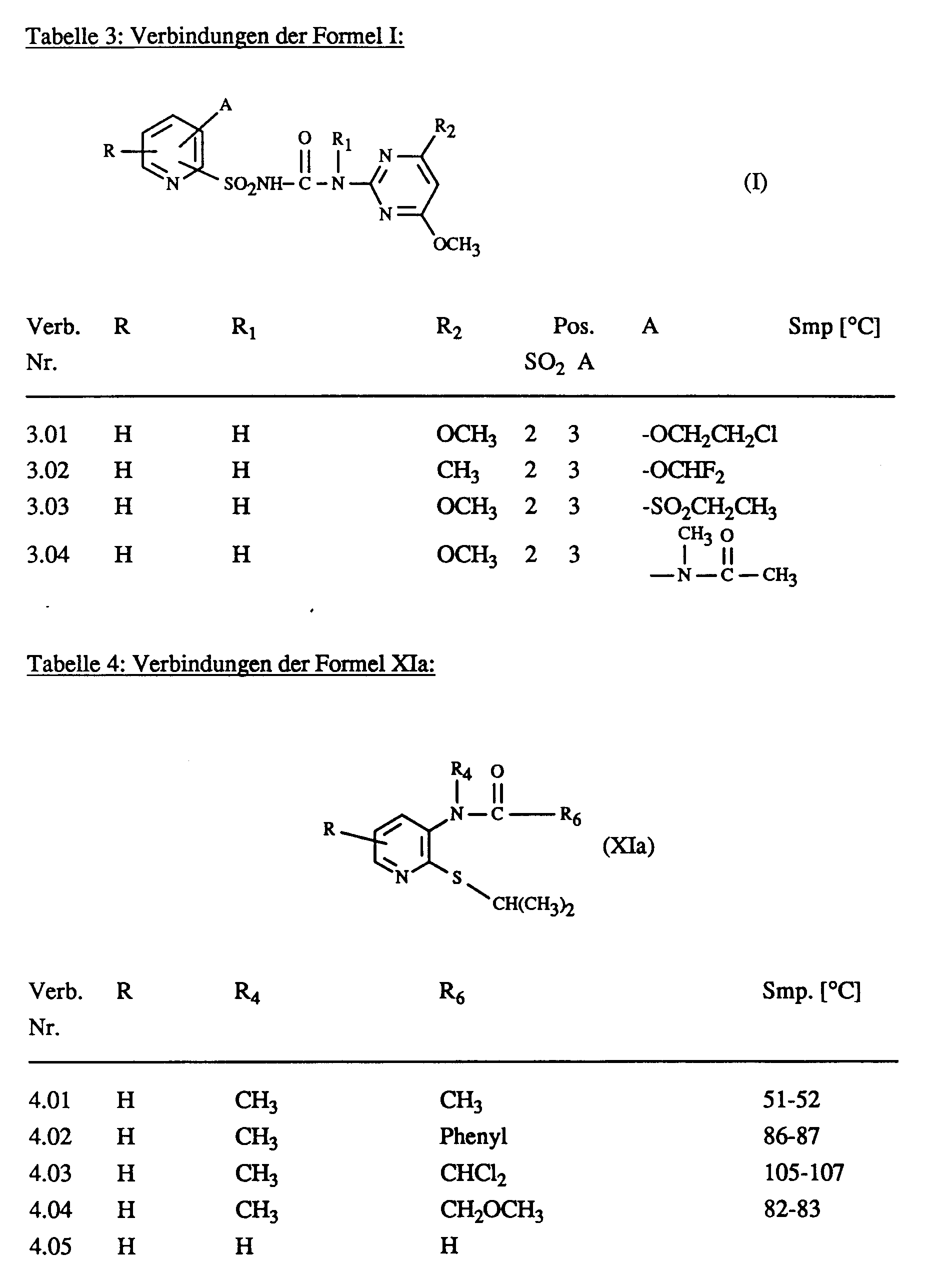

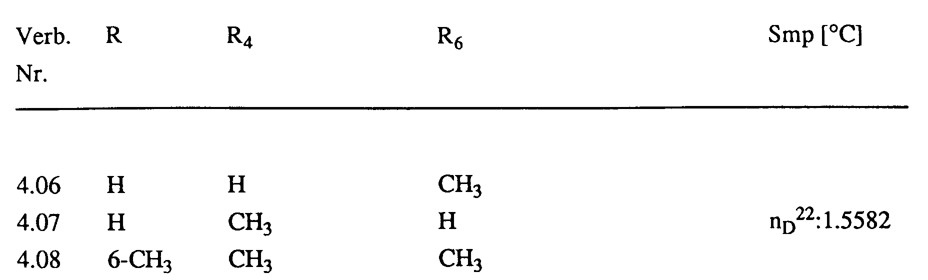

### Biologische Beispiele:

### Beispiel B1: Post-emergente phytotoxische Wirkungen der Herbizide der Formel I und der Mischungen Herbizid mit Safener der Formel II auf Mais

Unter Gewächshausbedingungen wird Mais in Kuntstofftöpfen bis zum 2,5-Blattstadium angezogen. In diesem Stadium wird ein Herbizid der Formel I allein sowie auch die Mischung des Herbizides mit Safenern der Formel II auf die Testpflanzen appliziert. Die Applikation erfolgt als wäßrige Suspension der Prüfsubstanzen mit 500 l Wasser/ha. Die Aufwandmengen für das Herbizid betragen 125/60/30/15/8 bzw. 250/125/60 g/ha, die Aufwandmengen für den Safener 60 g/ha. 23 Tage nach Applikation wird mit einer Prozentskala ausgewertet. 100 % bedeutet Testpflanze ist abgestorben, 0 % bedeutet keine phytotoxische Wirkung. Die Resultate zeigen, daß die eingesetzten Safener die durch das Herbizid auf Mais verursacht Schädigung deutlich reduzieren können. Beispiele für die gute Schutzwirkung der Safener sind in Tabelle B1 aufgeführt.

**Tabelle B1**

| Herbizid Verb. Nr. | Safener, g/ha | | Herbiziddosierung g/ha | | | | | |
|---|---|---|---|---|---|---|---|---|
| | | | 250 | 125 | 60 | 30 | 15 | 8 |
| 3.04 | - | | | 95 | 90 | 90 | 70 | 65 |
| 3.04 | IIb | 60 | | 5 | 0 | 0 | 0 | 0 |
| 3.03 | - | | 90 | 80 | 70 | | | |
| 3.03 | IIb | 60 | 50 | 30 | 10 | | | |

### Beispiel B2: Verwendung einer Mischung Herbizid der Formel I mit Safener der Formel II zur Samenbeizung bei Mais

Maissaatgut wird mit einem Safener angebeizt entsprechend einer Dosierung von 1 g/kg Saatgut. Anschließend wird der Mais unter Gewächshausbedingungen in Kunststofftöpfen bis zum 2,5-Blattstadium angezogen. Parallel zum behandelten Mais wird unbehandelter Mais bis zum selben Stadium kultiviert. In diesem Stadium wird das Herbizid der Formel I auf behandelte und unbehandelte Testpflanzen appliziert. Die Applikation erfolgt als wäßrige Suspension des Herbizids mit 500 l Wasser/ha. Die Aufwandmenge für das Herbizid beträgt 125, 60, 30, 15 bzw. 250/125/60 g/ha, die Aufwandmenge für den Saatbeizsafener der Formel II beträgt 1 g/kg Saatgut. 14 Tage nach Applikation wird mit einer Prozentskala ausgewertet. 100 % bedeutet Testpflanze ist abgestorben, 0 % bedeutet keine phtytotoxische Wirkung. Die Resultate zeigen, daß der Safener als Saatbeizmittel die durch das Herbizid postemergent verursachte Schädigung deutlich reduziert. Ähnliche Resultate werden erhalten, wenn das Herbizid preemergent appliziert wird. Beispiele für die gute Wirkung der Safener der Formel II gibt Tabelle B2:

**Tabelle B2**

| Herbizid Verb.Nr. | Safener, g/kg Saat | | Herbiziddosierung g/ha | | | | |
|---|---|---|---|---|---|---|---|
| | | | 250 | 125 | 60 | 30 | 15 |
| 3.04 | | | | 90 | 80 | 50 | |
| 3.04 | IIa | 1 | | 10 | 10 | 10 | |
| 3.02 | - | | | | 100 | 95 | 95 |
| 3.02 | IIa | 1 | | | 65 | 50 | 25 |
| 3.03 | - | | 90 | 80 | 70 | | |
| 3.03 | IIa | 1 | 0 | 0 | 0 | | |

### Beispiel B3: Post-emergente phytotoxische Wirkungen der Herbizide der Formel I und der Mischungen Herbizid mit Safener der Formel II in Zuckerrohr:

Unter Gewächshausbedingungen wird Zuckerrohr in Kuntstofftöpfen bis zum 3-Blattstadium angezogen. In diesem Stadium wird ein Herbizid der Formel I allein sowie auch die Mischung des Herbizides mit Safenern der Formel II auf die Testpflanzen appliziert. Die Applikation erfolgt als wäßrige Suspension der Prüfsubstanzen mit 500 l Wasser/ha. Die Aufwandmengen für das Herbizid betragen 120/60/30 g/ha, die Aufwandmengen für den Safener 60 g/ha. 21 Tage nach Applikation wird mit einer Prozentskala ausgewertet. 100 % bedeutet Testpflanze ist abgestorben, 0 % bedeutet keine phytotoxische Wirkung. Die Resultate zeigen, daß die eingesetzten Safener die durch das Herbizid auf Zuckerrohr verursachte Schädigung deutlich reduzieren können. Beispiele für die gute Schutzwirkung der Safener sind in Tabelle B3 aufgeführt:

**Tabelle B3**

| Herbizid Verb. Nr. | Safener, g/ha | | Herbiziddosierung g/ha | | |
|---|---|---|---|---|---|
| | | | 120 | 60 | 30 |
| 1.01 | - | | 50 | 30 | 15 |
| 1.01 | IIb | 60 | 30 | 0 | 0 |
| 1.01 | IIc | 60 | 25 | 5 | 0 |

### Beispiel B4: Herbizidwirkung vor dem Auflaufen der Pflanzen Kunststofftöpfe werden mit expandiertem

### Beispiel B1: Herbizidwirkung vor dem Auflaufen der Pflanzen

Kunststofftöpfe werden mit expandiertem Vermiculit gefüllt. Danach werden die Prüfsubstanzen so appliziert, daß die Endkonzentration im Nährmedium 70 ppm beträgt. Anschließend werden monokotyle und dikotyle Testpflanzen eingesät und unter Optimalbedingungen in der Klimakammer kultiviert Nach 10 Tagen wird mit einer neunstufigen Notenskala ausgewertet (1 = vollständige Schädigung, 9 = keine Wirkung). Boniturnoten von 1 bis 4 (insbesondere 1 bis 3) bedeuten eine gute bis sehr gute Herbizidwirkung. Beispiele für die gute herbizide Wirkung der Verbindungen der Formel Ib sind in Tabelle B4 aufgeführt:

**Tabelle B4**

| preemergente Wirkung von Herbiziden der Formel Ib: Konzentration der Wirkstoffemulsion: 70 ppm | | | | |
|---|---|---|---|---|
| Testpflanze: Wirkstoff Nr. | Nasturtium | Agrostis | Stellaria | Digitaria |
| 1.01 | 2 | 2 | 2 | 2 |
| 1.02 | 3 | 3 | 3 | 3 |
| 1.03 | 3 | 3 | 2 | 2 |
| 1.04 | 3 | 3 | 3 | 3 |
| 1.31 | 3 | 3 | 3 | 3 |
| 1.33 | 3 | 3 | 3 | 3 |
| 1.40 | 3 | 3 | 2 | 2 |
| 1.45 | 3 | 3 | 3 | 4 |
| 1.60 | 3 | 3 | 3 | 3 |
| 1.63 | 3 | 3 | 3 | 2 |
| 1.69 | 3 | 3 | 3 | 4 |
| 1.80 | 3 | 3 | 3 | 3 |
| 1.97 | 3 | 3 | 3 | 3 |
| 1.98 | 4 | 3 | 3 | 3 |
| 1.100 | 3 | 3 | 3 | 3 |
| 1.101 | 3 | 3 | 3 | 2 |

### Formulierungsbeispiele für Wirkstoffe der Formel II oder Mischungen derselben mit einem Herbizid der Formel I (% = Gewichtsprozent)

| 1. Emulsions-Konzentrate | a) | b) | c) |
|---|---|---|---|
| Wirkstoffmischung | 25 % | 40 % | 50 % |
| Ca-Dodecylbenzolsulfonat | 5 % | 8 % | 6 % |
| Ricinusöl-polyethylenglykolether (36 Mol EO) | 5 % | - | - |
| Tributylphenol-plyethylenglykolether (30 Mol EO) | - | 12 % | 4 % |
| Cyclohexanon | - | 15 % | 20 % |
| Xylolgemisch | 65 % | 25 % | 20 % |

Aus solchen Konzentrationen können durch Verdünnung mit Wasser Emulsionen jeder gewünschten Konzentration hergestellt werden.

| 2. Lösungen | a) | b) | c) | d) |
|---|---|---|---|---|
| Wirkstoffmischung | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxidiertes Kokosnußöl | - | - | 1 % | 5 % |
| Benzin (Siedegrenzen 160-190°) | - | - | 94 % | - |

Die Lösungen sind zur Anwendung in Form kleinster Tropfen geeignet.

| 3. Granulate | a) | b) |
|---|---|---|
| Wirkstoffmischung | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschließend im Vakuum abgedampft.

| 4. Stäubemittel | a) | b) |
|---|---|---|
| Wirkstoffmischung | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| 5. Spritzpulver | a) | b) | c) |
|---|---|---|---|
| Wirkstoffmischung | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mole EO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrate | |
|---|---|
| Wirkstoffmischung | 10 % |
| Octylphenolpolyethylenglykolether (4-5 Mol EO) | 3 % |
| Ca-Dodecylbenzolsulfonat | 3 % |
| Ricinusölpolyglykolether (35 Mol EO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoffmischung | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 8. Extruder-Granulate | |
|---|---|
| Wirkstoffmischung | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschließend im Luftstrom getrocknet.

| 9. Umhüllungs-Granulate | |
|---|---|
| Wirkstoffmischung | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

| 10. Suspensions-Konzentrate | |
|---|---|
| Wirkstoffmischung | 40 % |
| Ethylenglykol | 10 % |
| Nonylphenolpolyethylenglykolether (15 Mol EO) | 6 % |
| Na-Ligninsulfonat | 10 % |
| Carboxymethylcellulose | 1 % |
| 37%ige wäßrige Formaldehyd-Lösung | 0,2 % |
| Silikonöl in Form einer 75%igen wäßrigen Emulsion | 0,8 % |
| Wasser | 32 % |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Patentansprüche

1. Mittel zur selektiven Kontrolle von Unkräutern in Kulturen von Nutzpflanzen, dadurch gekennzeichnet, daß es neben inerten Trägern und Zusatzstoffen als Wirkstoff eine Mischung enthält, die
a) aus einer herbizid wirksamen Menge eines Pyridylsulfonylharnstoffes der Formel I 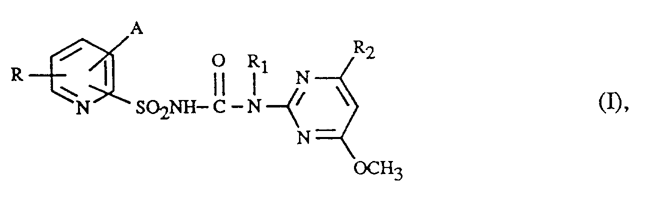 worin
R Wasserstoff, Halogen, C₁-C₄-Alkyl, C₁-C₄-Alkoxy oder C₁-C₄-Alkythio, oder durch Halogen ein- oder mehrfach substituiertes C₁-C₄-Alkyl;
R₁ Wasserstoff oder Methyl;
R₂ Methyl oder Methoxy;
A -X-R₃ oder-N-(R₄)R₅;
R₃ C₁-C₆-Alkyl, oder C₁-C₆-Alkyl welches ein- oder mehrfach durch Halogen, C₁-C₆-AIkoxy, C₃-C₆-Alkenyloxy, C₃-C₆-Alkinyloxy oder C₃-C₆-Cycloalkyl, welches seinerseits durch Sauerstoff unterbrochen sein kann, substituiert ist, C₃-C₆-Alkenyl, oder C₃-C₆-Alkenyl welches ein- oder mehrfach durch Halogen, C₁-C₆-Alkoxy, C₃-C₆-Alkenyloxy oder C₃-C₆-Alkinyloxy substituiert ist, C₄-C₆-Cycloalkyl, oder C₄-C₆-Cycloalkyl welches ein- oder mehrfach durch Halogen oder C₁-C₆-Alkoxy substituiert ist, oder C₃-C₆-Cycloalkyl, welches durch Sauerstoff unterbrochen ist, oder C₃-C₆-Alkinyl;
X Sauerstoff oder S(O)ₙ,
n 0,1 oder 2;
R₄ Wasserstoff, C₁-C₆-Alkyl, oder C₁-C₆-Alkyl welches ein- oder mehrfach durch Halogen, C₁-C₆-Alkoxy, C₃-C₆-Alkenyloxy, C₃-C₆-Alkinyloxy oder C₁-C₆-Alkylthio substituiert ist;
R₅ Wasserstoff, C₁-C₆-Alkyl, oder C₁-C₆-Alkyl welches ein- oder mehrfach durch Halogen, C₁-C₆-Alkoxy, C₃-C₆-Alkenyloxy, C₃-C₆-Alkinyloxy oder C₁-C₆-Alkylthio substituiert ist, oder C(O)R₆; und
R₆ Wasserstoff, C₁-C₆-Alkyl, C₃-C₆-Cycloalkyl, C₂-C₆-Alkinyl; oder C₁-C₆-Alkyl oder C₃-C₆-Cycloalkyl substituiert durch Halogen oder C₁-C₄-Alkoxy; C₂-C₆-Alkenyl oder C₂-C₆-Alkenyl substituiert durch Halogen; Phenyl, Benzyl, Naphtyl oder OR₁₂, oder Phenyl, Benzyl, Naphtyl substituiert durch Halogen, C₁-C₄-Alkyl, C₁-C₄-Halogenalkyl, C₁-C₄-Alkoxy, C₁-C₄-Halogenalkoxy, C₃-C₆-Alkenyloxy, C₃-C₆-Alkinyloxy, Nitro, Cyano, COOR₁₃, NR₁₅R₁₆, C(O)NR₁₇R₁₈, X₁R₂₀, SO₂NR₂₁R₂₂ oder X₂R₂₃;
R₁₂ C₁-C₆-Alkyl, C₁-C₆-Halogenalkyl, Oxetan-3-yl, C₄-C₆-Cycloalkyl, welches seinerseits durch Halogen, C₁-C₄-Alkyl oder C₁-C₄-Alkoxy substituiert sein kann; Phenyl, Benzyl, Naphtyl oder Phenyl, Benzyl, Naphtyl substituiert durch C₁-C4-Alkyl, C₁-C₄-Halogenalkyl, C₁-C₄-Alkoxy, C₁-C₄-Halogenalkoxy, C₁-C4-Alkylthio, C₁-C₄-Halogenalkylthio, C₁-C₄-Alkylsulfonyl, C₁-C₄-Alkylsulfinyl, Nitro, Cyano, COOR₂₇, NR₂₅R₂₆, CONR₂₈R₂₉ oder SO₂NR₃₀R₁₄; C₁-C₆-Alkyl substituiert durch C₁-C₄-Alkoxy, C₃-C₆-Cycloalkyl, Cyano, COOR₂₄ oder CONR₃₂R₃₃; C₃-C₆-Alkenyl, C₃-C₆-Halogenalkenyl, C₃-C₆-Alkinyl, C₃-C₆-Halogenalkinyl, X₃R₃₅ oder X₄R₃₆;
R₁₃ Wasserstoff, C₁-C₆-Alkyl, C₃-C₆-Alkenyl, C₃-C₆-Alkinyl oder Oxetan-3-yl;
R₁₄, R₁₅, R₁₆, R₁₇, R₁₈, R₂₁, R₂₂, R₂₅, R₂₆, R₂₈, R₂₉, R₃₀, R₃₂ und R₃₃ unabhängig voneinander Wasserstoff, C₁-C₄-Alkyl, C₃-C₆-Alkenyl oder C₃-C₆-Alkinyl; oder R₁₅ und R₂₅ unabhängig voneinander die Gruppen -C(O)-X₅-C₁-C₄-Alkyl oder -C(O)-C₁-C₄-Alkyl, welche ihrerseits durch Halogen substituiert sein können;
oder R₁₅ und R₁₆ oder R₁₇ und R₁₈ oder R₂₁ und R₂₂ oder R₂₅ und R₂₆ oder R₂₈ und R₂₉ oder R₃₀ und R₁₄ oder R₃₂ und R₃₃ zusammen für eine C₄-C₅-Alkylenkette, die seinerseits durch Sauerstoff oder NR₁₉ unterbrochen sein kann,
R₁₉ Wasserstoff, C₁-C₄-Alkyl, C₃-C₆-Alkenyl oder C₃-C₆-Alkinyl;
R₂₀ und R₃₅ unabhängig voneinander C₁-C₄-Alkyl oder C₁-C₄-Halogenalkyl;
R₂₃ und R₃₆ unabhängig voneinander C₁-C₄-Alkyl substituiert durch COOR₃₄;
R₂₄, R₂₇ und R₃₄ unabhängig voneinander Wasserstoff oder C₁-C₄-Alkyl;
X₁ und X₃ unabhängig voneinander Schwefel, SO oder SO₂;
X₂ und X₄ unabhängig voneinander Sauerstoff oder Schwefel;
X₅ Sauerstoff oder NR₃₇; und
R₃₇ Wasserstoff, C₁-C₄-Alkyl, C₃-C₆-Alkenyl oder C₃-C₆-Alkinyl bedeutet;
oder einem N-Oxid oder einem Salz der Verbindung der Formel I, und
b) aus einer herbizid-antagonistisch wirksamen Menge eines Sulfamoylphenylharnstoffes der Formel II 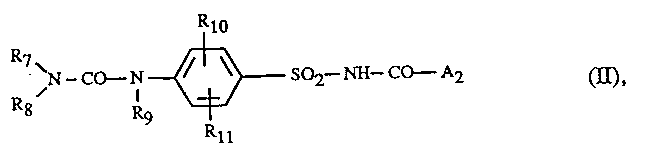 worin
A₂ für einen Rest aus der Gruppe 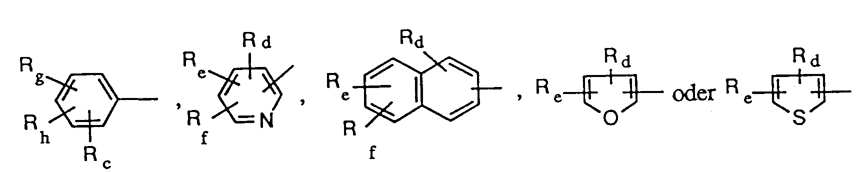 ist,
R₇ und R₈ unabhänig voneinander für Wasserstoff, C₁-C₈-Alkyl, C₃-C₈-Cycloalkyl, C₃-C₆-Alkenyl, C₃-C₆-Alkinyl, 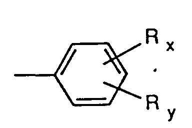 oder durch C₁-C₄-Alkoxy oder 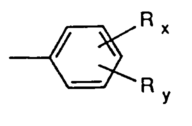 substituiertes C₁-C₄-Alkyl, oder
R₇ und R₈ gemeinsam für eine C₄-C₆-Alkylenbrücke oder eine durch Sauerstoff, Schwefel, SO, SO₂, NH oder N(C₁-C₄-Alkyl)- unterbrochene C₄-C₆-Alkylenbrücke stehen;
R₉ Wasserstoff oder C₁-C4-Alkyl ist;
R₁₀ und R₁₁ unabhängig voneinander für Wasserstoff, Halogen, Cyano, Nitro, Trifluormethyl, C₁-C₄-Alkyl C₁-C₄-Alkoxy, C₁-C₄-Alkylthio, C₁-C₄-Alkylsulfinyl, C₁-C₄-Alkylsulfonyl, -COORⱼ, CONRₖRₘ, -CORₙ, SO₂NRₖRₘ oder -OSO₂-C₁-C₄-Alkyl ; oder
R₁₀ und R₁₁ gemeinsam für eine C₃-C₄-Alkylenbrücke, die durch Halogen oder C₁-C₄-Alkyl substituiert sein kann, oder eine C₃-C₄-Alkenylenbrücke, die durch Halogen oder C₃ oder C₄-Alkyl substituiert sein kann, oder eine Butadienylenbrücke, die durch Halogen oder C₁-C₄-Alkyl substituiert sein kann, und
R_{g} und Rₕ unabhängig voneinander für Wasserstoff, Halogen, C₁-C₄-Alkyl, Trifluormethyl, Methoxy, Methylthio oder COORⱼ stehen; wobei
R_{c} Wasserstoff, Halogen, C₁-C₄-Alkyl oder Methoxy;
R_{d} Wasserstoff, Halogen, Nitro, C₁-C₄-Alkyl, C₁-C₄-Alkoxy, C₁-C₄-Alkythio, C₁-C₄-Alkylsulfinyl, C₁-C₄-Alkylsulfonyl, COORⱼ oder -CONRₖRₘ;
Rₑ Wasserstoff, Halogen, C₁-C₄-Halogenalkyl, C₁-C₄-Alkyl, -COORⱼ, Trifluormethyl oder Methoxy bedeuten; oder
R_{d} und Rₑ gemeinsam für eine C₃-C₄-Alkylenbrücke stehen;
R_{f} Wasserstoff, Halogen oder C₁-C₄-Alkyl ist;
Rₓ und R_{y} unabhängig voneinander Wasserstoff, Halogen, C₁-C₄-Alkyl, C₁-C₄-Alkoxy, C₁-C₄-Alkylthio, -COOR₁₇, Trifluormethyl, Nitro oder Cyano ist;
R₁₇ für Wasserstoff, C₁-C₁₀-Alkyl, C₁-C₄-Alkoxy-C₁-C₄-alkyl, C₁-C₄-Alkylthio-C₁-C₄-alkyl, Di-C₁-C₄-alkylamino-C₁-C₄-alkyl, Halogen-C₁-C₈-alkyl, C₂-C₈-Alkenyl, Halogen-C₂-C₈-alkenyl, C₃-C₈-Alkinyl, C₃-C₇-Cycloalkyl, Halogen-C₃-C₇-cycloalkyl, C₁-C₈-Alkylcarbonyl, Allylcarbonyl, C₃-C₇-Cycloalkylcarbonyl, Benzoyl, das unsubstituiert oder am Phenylring gleich oder verschieden bis zu dreifach durch Halogen, C₁-C₄-Alkyl, Halogen-C₁-C₄-alkyl, Halogen-C₁-C₄-alkoxy oder C₁-C₄-Alkoxy substituiert ist; oder Furoyl, Thienyl; oder C₁-C₄-Alkyl substituiert durch Phenyl, Halogenphenyl, C₁-C₄-AlkyIphenyl, C₁-C₄-Alkoxyphenyl, Halogen-C₁-C₄-alkylphenyl, Halogen-C₁-C₄-alkoxyphenyl, C₁-C₆-Alkoxycarbonyl, C₁-C₄-Alkoxy-C₁-C₈-alkoxycarbonyl, C₃-C₈-Alkenyloxycarbonyl, C₃-C₈-Alkinyloxycarbonyl, C₁-C₈-Alkylthiocarbonyl, C₃-C₈-Alkenylthiocarbonyl, C₃-C₈-Alkinylthiocarbonyl, Carbamoyl, Mono-C₁-C₄-alkylaminocarbonyl, Di-C₁-C₄-alkylaminocarbonyl; oder Phenylaminocarbonyl, das unsubstituiert oder am Phenyl gleich oder verschieden bis zu dreifach durch Halogen, C₁-C₄-Alkyl, Halogen-C₁-C₄-alkyl, Halogen-C₁-C₄-alkoxy oder C₁-C₄-Alkoxy oder einfach durch Cyano oder Nitro substituiert ist, oder Dioxolan-2-yl, das unsubstituiert ist oder durch ein oder zwei C₁-C₄-Alkylreste substituiert ist, oder Dioxan-2-yl, das unsubstituiert ist oder durch ein oder zwei C₁-C₄-Alkylreste substituiert ist, oder C₁-C₄-Alkyl, das durch Cyano, Nitro, Carboxyl oder C₁-C₈-Alkylthio-C₁-C₈-alkoxycarbonyl substituiert ist, steht;
Rⱼ, Rₖ und Rₘ unabhängig voneinander Wasserstoff oder C₁-C₄-Alkyl ist;
Rₖ und Rₘ gemeinsam eine C₄-C₆-Alkylenbrücke oder eine durch Sauerstoff, NH oder -N(C₁-C₄-Alkyl)- unterbrochene C₄-C₆-Alkylenbrücke bilden; und
Rₙ C₁-C₄-Alkyl, Phenyl oder durch Halogen, C₁-C₄-Alkyl, Methoxy, Nitro oder Trifluormethyl substituiertes Phenyl ist;
oder einem Salz der Verbindung der Formel II, besteht.

2. Mittel nach Anspruch 1, dadurch gekennzeichnet, daß
R₆ Wasserstoff, C₁-C₆-Alkyl oder C₃-C₆-Cycloalkyl bedeutet.

3. Mittel nach Anspruch 1, dadurch gekennzeichnet, daß
R₃ C₁-C₆-Alkyl, oder C₁-C₆-Alkyl welches ein- oder mehrfach durch Halogen, C₁-C₆-Alkoxy, C₃-C₆-Alkenyloxy, C₃-C₆-Alkinyloxy oder C₃-C₆-Cycloalkyl, welches seinerseits durch Sauerstoff unterbrochen sein kann, substituiert ist, C₃-C₆-Alkenyl, oder C₃-C₆-Alkenyl welches ein- oder mehrfach durch Halogen, C₁-C₆-Alkoxy, C₃-C₆-Alkenyloxy oder C₃-C₆-Alkinyloxy substituiert ist, C₄-C₆-Cycloalkyl, oder C₄-C₆-Cycloalkyl welches ein- oder mehrfach durch Halogen oder C₁-C₆-Alkoxy substituiert ist oder durch Sauerstoff unterbrochen sein kann, oder C₃-C₆-Alkinyl; und
R₆ Wasserstoff oder C₁-C₆-Alkyl bedeutet.

4. Mittel nach Anspruch 1, dadurch gekennzeichnet, daß R₆ für Wasserstoff, C₁-C₆-Alkyl, C₃-C₆-Cycloalkyl, C₂-C₆-Alkinyl; oder C₁-C₆-Alkyl oder C₃-C₆-Cycloalkyl substituiert durch Halogen oder C₁-C₄-Alkoxy; C₂-C₆-Alkenyl oder C₂-C₆-Alkenyl substituiert durch Halogen steht.

5. Mittel nach Anspruch 1, enthaltend eine Verbindung der Formel Ia 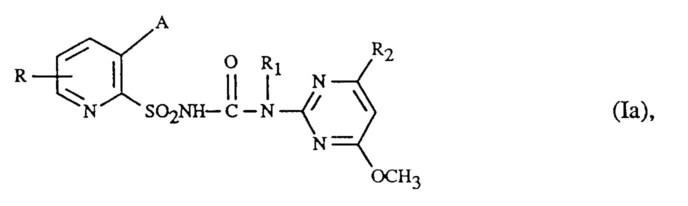 worin A, R, R₁, R₂ und R₃ die in Anspruch 1 angegebenen Bedeutungen haben.

6. Mittel nach Anspruch 5, worin R Wasserstoff bedeutet.

7. Mittel nach Anspruch 5, worin A -X-R₃ bedeutet.

8. Mittel nach Anspruch 5, worin A -N-(R₄)R₅ bedeutet.

9. Mittel nach Anspruch 5, worin R₅ C(O)R₆ bedeutet.

10. Mittel nach Anspruch 5, worin R und R₁ Wasserstoff und A Difluormethoxy oder die Gruppe 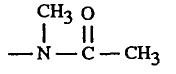 bedeuten.

11. Mittel nach Anspruch 6, worin R und R₁ Wasserstoff und A die Gruppe 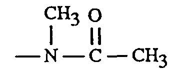 oder -SO₂-C₁-C₄-Alkyl bedeuten.

12. Mittel nach Anspruch 1, worin in den Verbindungen der Formel II
A₂ ein Rest der Formel 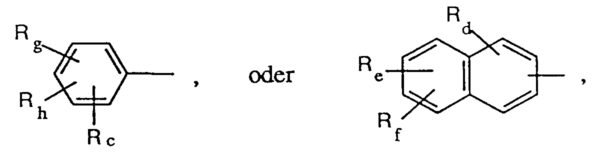 worin
R_{g} und Rₕ unabhängig voneinander für Wasserstoff, Halogen, C₁-C₄-Alkyl, Trifluormethyl, Methoxy, Methylthio oder COORⱼ stehen, wobei
R_{c} Wasserstoff, Halogen, C₁-C₄-Alkyl oder Methoxy,
R_{d} Wasserstoff, Halogen, Nitro, C₁-C₄-Alkyl, C₁-C₄-Alkoxy, C₁-C₄-Alkylthio, C₁-C₄-Alkysulfinyl, C₁-C₄-Alkylsulfonyl, -COORⱼ oder -CONRₖRₘ,
Rₑ Wasserstoff, Halogen, C₁-C₄-Alkyl, -COORⱼ, Trifluormethyl oder Methoxy ist, oder R_{d} und Rₑ gemeinsam für eine C₃ oder C₄-Alkylenbrücke bilden,
R_{f} Wasserstoff, Halogen oder C₁-C₄-Alkyl ist,
Rⱼ, Rₖ und Rₘ unabhängig voneinander Wasserstoff oder C₁-C₄-Alkyl sind, und
Rₖ und Rₘ gemeinsam eine C₄-C₆-Alkylenbrücke oder eine durch Sauerstoff, NH oder -N(C₁-C₄-Alkyl)- unterbrochene C₄-C₆-Alkylenbrücke bilden.

13. Mittel nach Anspruch 12, worin in den Verbindungen der Formel II R_{c}, R_{d}, Rₑ, R_{g} und Rₕ unabhängig voneinander Wasserstoff, C₁-C₄-Alkyl oder Methoxy sind

14. Mittel nach Anspruch 1, worin in den Verbindungen der Formel II R₁₀ und R₁₁ Wasserstoff sind

15. Mittel nach Anspruch 5, enthaltend eine Verbindung der Formel IIf 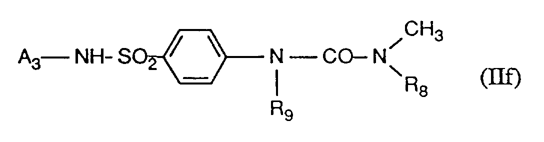 worin R₈ und R₉ die unter Formel II in Anspruch 1 angegebenen Bedeutung haben und A₃ für die Gruppen 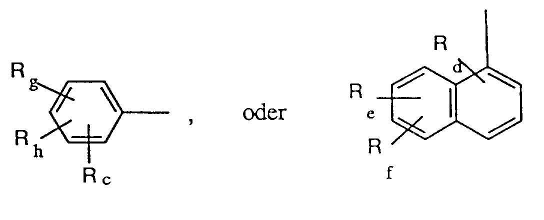 steht.

16. Mittel nach Anspruch 15, worin in den Verbindungen der Formel IIf R₈ und R₉ Wasserstoff oder Methyl sind

17. Mittel nach Anspruch 1, worin in den Verbindungen der Formel II R₉, R₁₀ und R₁₁ Wasserstoff sind.

18. Mittel nach Anspruch 5, dadurch gekennzeichnet, daß es als Verbindung der Formel II eine Verbindung der Formel IIa 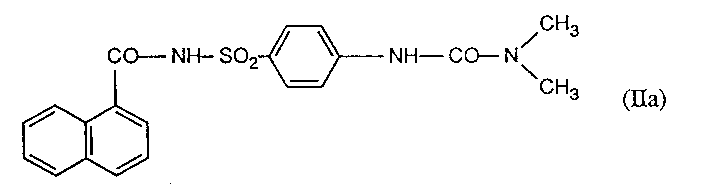 oder der Formel IIb 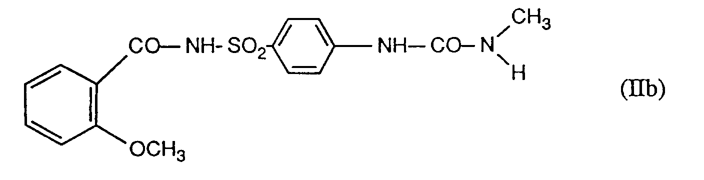 oder der Formel IIc 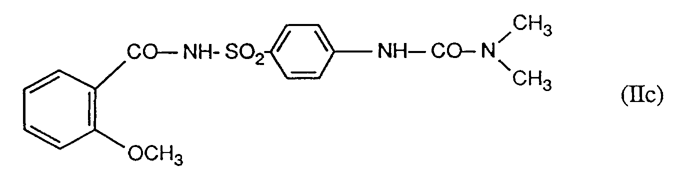 oder der Formel IId 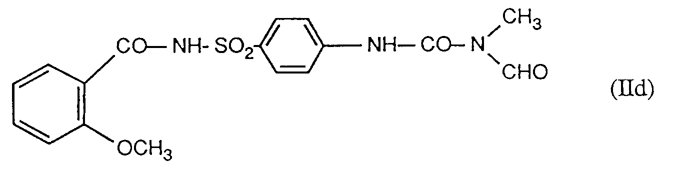 oder der Formel IIe 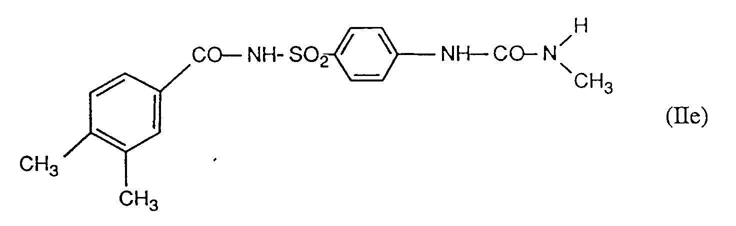 enthält.

19. Verfahren zum selektiven Bekämpfen von Unkräutern und Gräsern in Nutzpflanzenkulturen, dadurch gekennzeichnet, daß man die Kulturen, deren Saatgut oder deren Anbaufläche gleichzeitig oder unabhängig voneinander mit einer wirksamen Menge eines Herbizides der Formel I gemäß Anspruch 1 und einer herbizid-antagonistisch wirksamen Menge einer Verbindung der Formel II gemäß Anspruch 1 behandelt.

20. Verfahren gemäß Anspruch 19, dadurch gekennzeichnet, daß man Kulturpflanzenbestände oder Anbauflächen für Kulturpflanzen mit 0,001 bis 2 kg/ha einer Verbindung der Formel I gemäß Anspruch 1 und einer Menge von 0,005 bis 0,5 kg/ha einer Verbindung der Formel II gemäß Anspruch 1 behandelt.

21. Verfahren gemäß Anspruch 19 zum selektiven Bekämpfen von Unkräutern und Gräsern in Kulturen von Mais und Zuckerrohr.

## Claims

1. A composition for the selective control of weeds in crops of useful plants, which comprises, as active ingredient, together with inert carriers and adjuvants, a mixture comprising
a) a herbicidally effective amount of a pyridylsulfonylurea of formula I 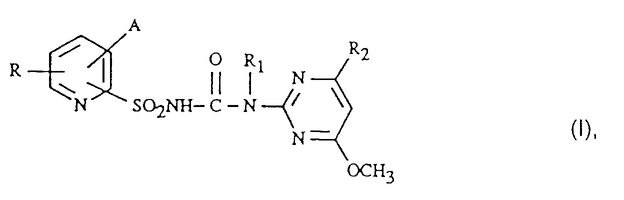 wherein
R is hydrogen, halogen, C₁-C₄alkyl, C₁-C₄alkoxy or C₁-C₄alkylthio, or is C₁-C₄alkyl mono- or poly-substituted by halogen;
R₁ is hydrogen or methyl;
R₂ is methyl or methoxy;
A is -X-R₃ or -N-(R₄)R₅;
R₃ is C₁-C₆alkyl, or C₁-C₆alkyl that is mono- or poly-substituted by halogen, C₁-C₆-alkoxy, C₃-C₆alkenyloxy, C₃-C₆alkynyloxy or by C₃-C₆cycloalkyl, which may for its part be interrupted by oxygen; or is C₃-C₆alkenyl, or C₃-C₆alkenyl that is mono- or poly-substituted by halogen, C₁-C₆alkoxy, C₃-C₆alkenyloxy or by C₃-C₆alkynyloxy; or is C₄-C₆cycloalkyl, or C₄-C₆cycloalkyl that is mono- or poly-substituted by halogen or by C₁-C₆alkoxy; or is C₃-C₆cycloalkyl that is interrupted by oxygen; or is C₃-C₆alkynyl;
X is oxygen or S(O)ₙ;
n is 0,1 or 2;
R₄ is hydrogen, C₁-C₆alkyl, or C₁-C₆alkyl that is mono- or poly-substituted by halogen, C₁-C₆alkoxy, C₃-C₆alkenyloxy, C₃-C₆alkynyloxy or by C₁-C₆alkylthio;
R₅ is hydrogen, C₁-C₆alkyl, or C₁-C₆alkyl that is mono- or poly-substituted by halogen, C₁-C₆alkoxy, C₃-C₆alkenyloxy, C₃-C₆alkynyloxy or by C₁-C₆alkylthio, or is C(O)R₆; and
R₆ is hydrogen, C₁-C₆alkyl, C₃-C₆cycloalkyl or C₂-C₆alkynyl; or is C₁-C₆alkyl or C₃-C₆-cycloalkyl each substituted by halogen or by C₁-C₄alkoxy; or is C₂-C₆alkenyl, or C₂-C₆-alkenyl substituted by halogen; or is phenyl, benzyl, naphthyl or OR₁₂ ,or phenyl, benzyl or naphthyl each substituted by halogen, C₁-C₄alkyl, C₁-C₄haloalkyl, C₁-C₄-alkoxy, C₁-C₄haloalkoxy, C₃-C₆alkenyloxy, C₃-C₆alkynyloxy, nitro, cyano, COOR₁₃, NR₁₅R₁₆, C(O)NR₁₇R₁₈, X₁R₂₀, SO₂NR₂₁R₂₂ or by X₂R₂₃;
R₁₂ is C₁-C₆alkyl, C₁-C₆haloalkyl, oxetan-3-yl, or C₄-C₆cycloalkyl, which may for its part be substituted by halogen, C₁-C₄alkyl or by C₁-C₄alkoxy; or is phenyl, benzyl or naphthyl, or phenyl, benzyl or naphthyl each substituted by C₁-C₄alkyl, C₁-C₄haloalkyl, C₁-C₄alkoxy, C₁-C₄haloalkoxy, C₁-C₄alkylthio, C₁-C₄haloalkylthio, C₁-C₄alkylsulfonyl, C₁-C₄alkylsulfinyl, nitro, cyano, COOR₂₇, NR₂₅R₂₆, CONR₂₈R₂₉ or by SO₂NR₃₀R₁₄; or is C₁-C₆alkyl substituted by C₁-C₄alkoxy, C₃-C₆cycloalkyl, cyano, COOR₂₄ or by CONR₃₂R₃₃; or is C₃-C₆alkenyl, C₃-C₆haloalkenyl, C₃-C₆alkynyl, C₃-C₆haloalkynyl, X₃R₃₅ or X₄R₃₆;
R₁₃ is hydrogen, C₁-C₆alkyl, C₃-C₆alkenyl, C₃-C₆alkynyl or oxetan-3-yl;
R₁₄, R₁₅, R₁₆, R₁₇, R₁₈, R₂₁, R₂₂, R₂₅, R₂₆, R₂₈, R₂₉, R₃₀, R₃₂ and R₃₃ are each independently of the others hydrogen, C₁-C₄alkyl, C₃-C₆alkenyl or C₃-C₆alkynyl; or R₁₅ and R₂₅ are each independently of the other the group -C(O)-X₅-C₁-C₄alkyl or -C(O)-C₁-C₄alkyl, which may for their part be substituted by halogen; or R₁₅ and R₁₆ or R₁₇ and R₁₈ or R₂₁ and R₂₂ or R₂₅ and R₂₆ or R₂₈ and R₂₉ or R₃₀ and R₁₄ or R₃₂ and R₃₃ together form a C₄-C₅alkylene chain, which may for its part be interrupted by oxygen or by NR₁₉;
R₁₉ is hydrogen, C₁-C₄alkyl, C₃-C₆alkenyl or C₃-C₆alkynyl;
R₂₀ and R₃₅ are each independently of the other C₁-C₄alkyl or C₁-C₄haloalkyl;
R₂₃ and R₃₆ are each independently of the other C₁-C₄alkyl substituted by COOR₃₄;
R₂₄, R₂₇ and R₃₄ are each independently of the other hydrogen or C₁-C₄alkyl;
X₁ and X₃ are each independently of the other sulfur, SO or SO₂;
X₂ and X₄ are each independently of the other oxygen or sulfur;
X₅ is oxygen or NR₃₇; and
R₃₇ is hydrogen, C₁-C₄alkyl, C₃-C₆alkenyl or C₃-C₆alkynyl;
or an N-oxide or a salt of a compound of formula I, and
b) a herbicide-antagonistically effective amount of a sulfamoylphenylurea of formula II 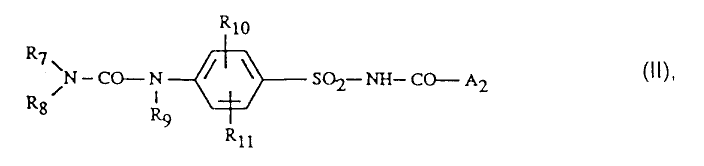 wherein
A₂ is a radical from the group 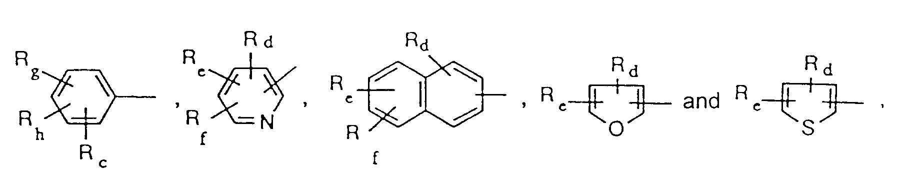
R₇ and R₈ are each independently of the other hydrogen, C₁-C₈alkyl, C₃-C₈cycloalkyl, C₃-C₆alkenyl, C₃-C₆alkynyl or 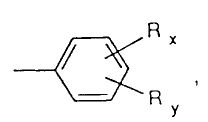 or are C₁-C₄alkyl substituted by C₁-C₄alkoxy or by 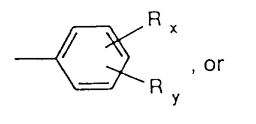
R₇ and R₈ together form a C₄-C₆alkylene bridge, or a C₄-C₆alkylene bridge interrupted by oxygen, sulfur, SO, SO₂, NH or by -N(C₁-C₄alkyl)-;
R₉ is hydrogen or C₁-C₄alkyl;
R₁₀ and R₁₁ are each independently of the other hydrogen, halogen, cyano, nitro, trifluoromethyl, C₁-C₄alkyl, C₁-C₄alkoxy, C₁-C₄alkylthio, C₁-C₄alkylsulfinyl, C₁-C₄alkyl-sulfonyl, `COORⱼ, CONRₖRₘ, -CORₙ, -SO₂NRₖRm or -OSO₂-C₁-C₄alkyl; or
R₁₀ and R₁₁ together form a C₃-C₄alkylene bridge, which may be substituted by halogen or by C₁-C₄alkyl, or a C₃-C₄alkenylene bridge, which may be substituted by halogen or by C₃- or C₄-alkyl, or a butadienylene bridge, which may be substituted by halogen or by C₁-C₄alkyl, and
R_{g} and Rₕ are each independently of the other hydrogen, halogen, C₁-C₄alkyl, trifluoromethyl, methoxy, methylthio or -COORⱼ;
R_{c} is hydrogen, halogen, C₁-C₄alkyl or methoxy;
R_{d} is hydrogen, halogen, nitro, C₁-C₄alkyl, C₁-C₄alkoxy, C₁-C₄alkylthio, C₁-C₄alkyl-sulfinyl, C₁-C₄alkylsulfonyl, -COORⱼ or -CONRₖRₘ; and
Rₑ is hydrogen, halogen, halo-C₁-C₄alkyl, C₁-C₄alkyl, -COORⱼ, trifluoromethyl or methoxy; or
R_{d} and Rₑ together form a C₃-C₄alkylene bridge;
R_{f} is hydrogen, halogen or C₁-C₄alkyl;
Rₓ and R_{y} are each independently of the other hydrogen, halogen, C₁-C₄alkyl, C₁-C₄-alkoxy, C₁-C₄alkylthio, -COOR₁₇, trifluoromethyl, nitro or cyano;
R₁₇ is hydrogen, C₁-C₁₀alkyl, C₁-C₄alkoxy-C₁-C₄alkyl, C₁-C₄alkylthio-C₁-C₄alkyl, di-C₁-C₄alkylamino-C₁-C₄alkyl, halo-C₁-C₈alkyl, C₂-C₈alkenyl, halo-C₂-C₈alkenyl, C₃-C₈-alkynyl, C₃-C₇cycloalkyl, halo-C₃-C₇cycloalkyl, C₁-C₈alkylcarbonyl, allylcarbonyl, C₃-C₇-cycloalkylcarbonyl, benzoyl that is unsubstituted or mono- to tri-substituted in the phenyl ring by the same or different substituents selected from halogen, C₁-C₄alkyl, halo-C₁-C₄alkyl, halo-C₁-C₄alkoxy and C₁-C₄alkoxy; or is furoyl or thienyl; or is C₁-C₄-alkyl substituted by phenyl, halophenyl, C₁-C₄alkylphenyl, C₁-C₄alkoxyphenyl, halo-C₁-C₄alkylphenyl, halo-C₁-C₄alkoxyphenyl, C₁-C₆alkoxycarbonyl, C₁-C₄alkoxy-C₁-C₈-alkoxycarbonyl, C₃-C₈alkenyloxycarbonyl, C₃-C₈alkynyloxycarbonyl, C₁-C₈alkylthio-carbonyl, C₃-C₈alkenylthiocarbonyl, C₃-C₈alkynylthiocarbonyl, carbamoyl, mono-C₁-C₄alkylaminocarbonyl or by di-C₁-C₄alkylaminocarbonyl; or is phenylaminocarbonyl that is unsubstituted or mono- to tri-substituted in the phenyl ring by the same or different substituents selected from halogen, C₁-C₄alkyl, halo-C₁-C₄alkyl, halo-C₁-C₄alkoxy and C₁-C₄alkoxy or that is mono-substituted in the phenyl ring by cyano or by nitro; or is dioxolan-2-yl that is unsubstituted or substituted by one or two C₁-C₄alkyl radicals; or is dioxan-2-yl that is unsubstituted or substituted by one or two C₁-C₄alkyl radicals; or is C₁-C₄alkyl that is substituted by cyano, nitro, carboxy or by C₁-C₈alkyl-thio-C₁-C₈alkoxycarbonyl;
Rⱼ, Rₖ and Rₘ are each independently of the others hydrogen or C₁-C₄alkyl; or
Rₖ and Rₘ together form a C₄-C₆alkylene bridge, or a C₄-C₆alkylene bridge interrupted by oxygen, NH or by -N(C₁-C₄alkyl)-; and
Rₙ is C₁-C₄alkyl, phenyl, or phenyl substituted by halogen, C₁-C₄alkyl, methoxy, nitro or by trifluoromethyl;
or a salt of a compound of formula II.

2. A composition according to claim 1, wherein
R₆ is hydrogen, C₁-C₆alkyl or C₃-C₆cycloalkyl.

3. A composition according to claim 1, wherein
R₃ is C₁-C₆alkyl, or C₁-C₆alkyl that is mono- or poly-substituted by halogen, C₁-C₆-alkoxy, C₃-C₆alkenyloxy, C₃-C₆alkynyloxy or by C₃-C₆cycloalkyl, which may for its part be interrupted by oxygen; or is C₃-C₆alkenyl, or C₃-C₆alkenyl that is mono- or poly-substituted by halogen, C₁-C₆alkoxy, C₃-C₆alkenyloxy or by C₃-C₆alkynyloxy; or is C₄-C₆cycloalkyl, or C₄-C₆cycloalkyl that is mono- or poly-substituted by halogen or by C₁-C₆alkoxy or may be interrupted by oxygen; or is C₃-C₆alkynyl; and
R₆ is hydrogen or C₁-C₆alkyl.

4. A composition according to claim 1, wherein R₆ is hydrogen, C₁-C₆alkyl, C₃-C₆cyclo-alkyl or C₂-C₆alkynyl; or is C₁-C₆alkyl or C₃-C₆cycloalkyl each substituted by halogen or by C₁-C₄alkoxy; or is C₂-C₆alkenyl, or C₂-C₆alkenyl substituted by halogen.

5. A composition according to claim 1, comprising a compound of formula Ia 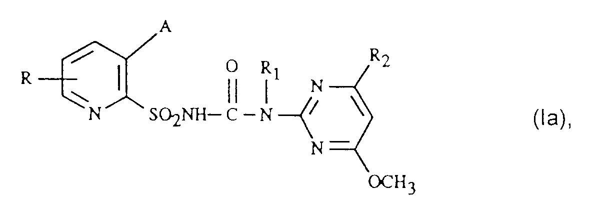 wherein A, R, R₁, R₂ and R₃ are as defined in claim 1.

6. A composition according to claim 5, wherein R is hydrogen.

7. A composition according to claim 5, wherein A is -X-R₃.

8. A composition according to claim 5, wherein A is -N-(R₄)R₅.

9. A composition according to claim 5, wherein R₅ is C(O)R₆.

10. A composition according to claim 5, wherein each of R and R₁ is hydrogen and A is difluoromethoxy or the group

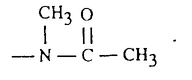

11. A composition according to claim 6, wherein each of R and R₁ is hydrogen and A is the group 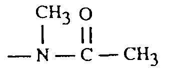 or -SO₂-C₁-C₄alkyl.

12. A composition according to claim 1, wherein, in the compound of formula II, A₂ is a radical of the formula 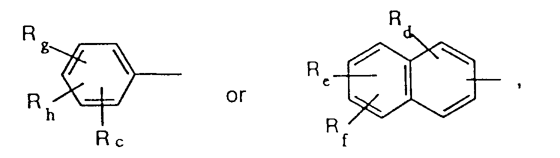 wherein
R_{g} and Rₕ are each independently of the other hydrogen, halogen, C₁-C₄alkyl, trifluoromethyl, methoxy, methylthio or COORⱼ;
R_{c} is hydrogen, halogen, C₁-C₄alkyl or methoxy;
R_{d} is hydrogen, halogen, nitro, C₁-C₄alkyl, C₁-C₄alkoxy, C₁-C₄alkylthio, C₁-C₄alkyl-sulfinyl, C₁-C₄alkylsulfonyl, COORⱼ or CONRₖRₘ; and
Rₑ is hydrogen, halogen, C₁-C₄alkyl, -COORⱼ,trifluoromethyl or methoxy; or
R_{d} and Rₑ together form a C₃- or C₄-alkylene bridge;
R_{f} is hydrogen, halogen or C₁-C₄alkyl;
Rⱼ, Rₖ, and Rₘ are each independently of the others hydrogen or C₁-C₄alkyl; or
Rₖ and Rₘ together form a C₄-C₆alkylene bridge, or a C₄-C₆alkylene bridge interrupted by oxygen, NH or by -N(C₁-C₄alkyl)-.

13. A composition according to claim 12, wherein, in the compound of formula II, R_{c}, R_{d}, Rₑ, R_{g} and Rₕ are each independently of the others hydrogen, C₁-C₄alkyl or methoxy.

14. A composition according to claim 1, wherein, in the compound of formula II, each of R₁₀ and R₁₁ is hydrogen.

15. A composition according to claim 5, comprising a compound of formula IIf 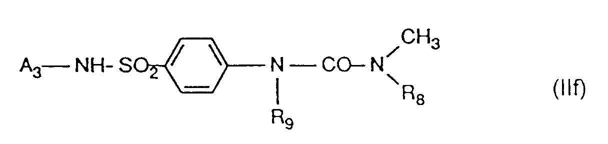 wherein R₈ and R₉ are as defined under formula II in claim 1 and A₃ is the group

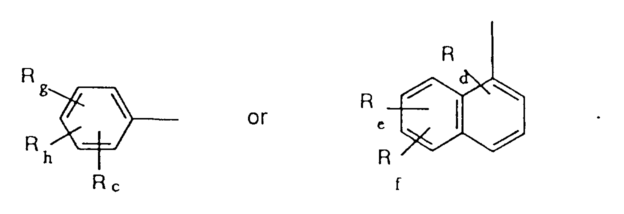

16. A composition according to claim 15, wherein, in the compound of formula IIf, each of R₈ and R₉ is hydrogen or methyl.

17. A composition according to claim 1, wherein, in the compound of formula II, each of R₉, R,₀ and R₁₁ is hydrogen.

18. A composition according to claim 5, which comprises as compound of formula II a compound of formula IIa 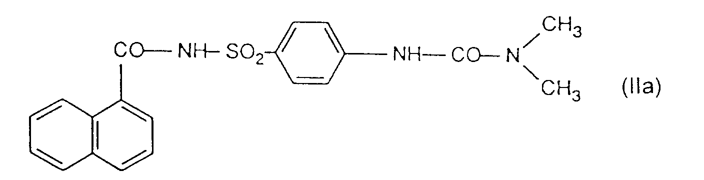 or of formula IIb 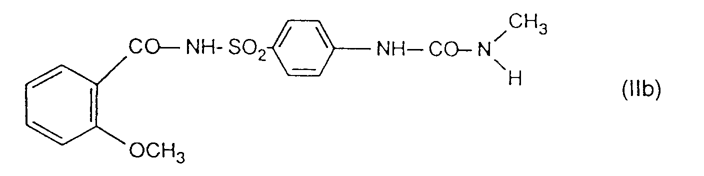 or of formula IIc 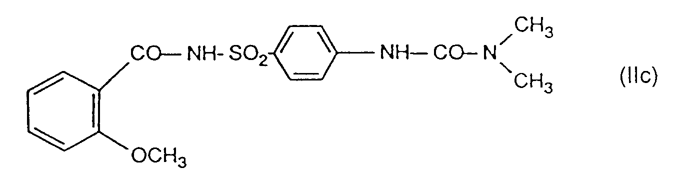 or of formula IId 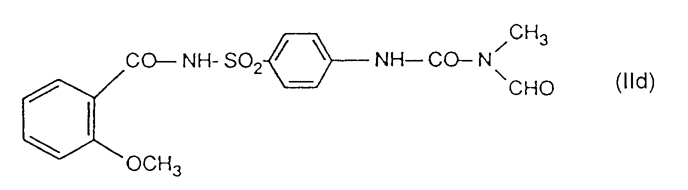 or of formula IIe

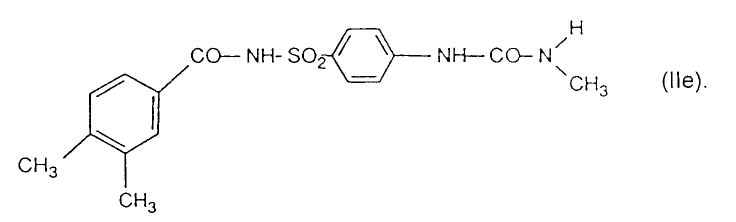

19. A method of selectively controlling weeds and grasses in crops of useful plants, which comprises treating the crops, their seed or the crop area thereof with an effective amount of a herbicide of formula I according to claim 1 and a herbicide-antagonistically effective amount of a compound of formula II according to claim 1, simultaneously or independently of one another.

20. A method according to claim 19, which comprises treating cultivated plant crops or crop areas for cultivated plants with from 0.001 to 2 kg/ha of a compound of formula I according to claim 1 and an amount of from 0.005 to 0.5 kg/ha of a compound of formula II according to claim 1.

21. A method according to claim 19 of selectively controlling weeds and grasses in crops of maize and sugar cane.

## Revendications

1. Un agent pour le contrôle sélectif des mauvaises herbes dans les cultures de plantes utiles, caractérisé en ce que, outre des supports et des additifs, il contient comme matière active un mélange qui est constitué
a) d'une quantité efficace du point de vue herbicide d'une pyridylsulfonylurée de formule I 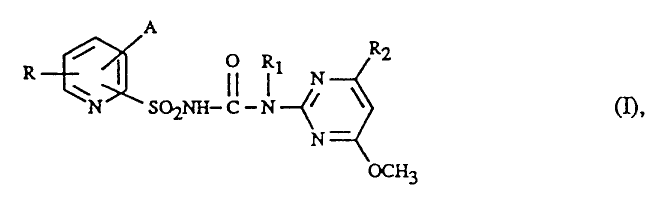 où
R signifie l'hydrogène, un halogène, un groupe C₁-C₄-alkyle, C₁-C₄-alcoxy ou C₁-C₄-alkylthio ou un groupe C₁-C₄-alkyle mono- ou polysubstitué par un halogène,
R₁ signifie l'hydrogène ou un groupe méthyle;
R₂ signifie un groupe méthyle ou méthoxy;
A signifie -X-R₃ ou -N-(R₄)R₅;
R₃ signifie un groupe C₁-C₆-alkyle ou C₁-C₆-alkyle qui est mono- ou polysubstitué par un halogène, un groupe C₁-C₆-alcoxy, C₃-C₆-alcényloxy, C₃-C₆-alcynyloxy ou C₃-C₆-cycloalkyle, qui de son côté peut être interrompu par de l'oxygène, un groupe C₃-C₆-alcényle ou C₃-C₆-alcényle qui est mono- ou polysubstitué par un halogène, un groupe C₁-C₆-alcoxy, C₃-C₆-alcényloxy ou C₃-C₆-alcynyloxy, C₄-C₆-cycloalkyle ou C₄-C₆-cycloalkyle qui est mono- ou polysubstitué par un halogène ou un groupe C₁-C₆-alcoxy ou bien C₃-C₆-cycloalkyle qui est interrompu par de l'oxygène ou un groupe C₃-C₆-alcynyle;
X signifie de l'oxygène ou S(O)ₙ;
n signifie 0, 1 ou 2;
R₄ signifie l'hydrogène, un groupe C₁-C₆-alkyle ou C₁-C₆-alkyle qui est mono- ou polysubstitué par un halogène, un groupe C₁-C₆-alcoxy, C₃-C₆-alcényloxy, C₃-C₆-alcynyloxy ou C₁-C₆-alkylthio;
R₅ signifie l'hydrogène, un groupe C₁-C₆-alkyle ou C₁-C₆-alkyle qui est mono- ou polysubstitué par un halogène, un groupe C₁-C₆-alcoxy, C₃-C₆-alcényloxy, C₃-C₆-alcynyloxy ou C₁-C₆-alkylthio, ou bien C(O)R₆; et
R₆ signifie l'hydrogène, un groupe C₁-C₆-alkyle, C₃-C₆-cycloalkyle, C₂-C₆-alcynyle; ou bien C₁-C₆-alkyle ou C₃-C₆-cycloalkyle substitué par un halogène ou par un groupe C₁-C₄-alcoxy; C₂-C₆-alcényle ou C₂-C₆-alcényle substitué par un halogène; phényle, benzyle, naphtyle ou OR₁₂, ou bien phényle, benzyle, napthyle substitués par un halogène, un groupe C₁-C₄-alkyle, C₁-C₄-halogènealkyle, C₁-C₄-alcoxy, C₁-C₄-halogènealcoxy, C₃-C₆-alcényloxy, C₃-C₆-alcynyloxy, nitro, cyano, COOR₁₃, NR₁₅R₁₆, C(O)NR₁₇R₁₈, X₁R₂₀, SO₂NR₂₁R₂₂ ou X₂R₂₃;
R₁₂ signifie un groupe C₁-C₆-alkyle, C₁-C₆-halogènealkyle, 1,3-époxy-3-propyle, C₄-C₆-cycloalkyle, qui de son côté peut être substitué par un halogène, un groupe C₁-C₄-alkyle ou C₁-C₄-alcoxy; phényle, benzyle, naphtyle ou phényle, benzyle, napthyle substitués par un groupe C₁-C₄-alkyle, C₁-C₄-halogènealkyle, C₁-C₄-alcoxy, C₁-C₄-halogènealcoxy, C₁-C₄-alkylthio, C₁-C₄-halogènealkylthio, C₁-C₄-alkylsulfonyle, C₁-C₄-alkylsulfinyle, nitro, cyano, COOR₂₇, NR₂₅R₂₆, CONR₂₈R₂₉ ou SO₂NR₃₀R₁₄; C₁-C₆-alkyle substitué par un groupe C₁-C₄-alcoxy, C₃-C₆-cycloalkyle, cyano, COOR₂₄ ou CONR₃₂R₃₃; C₃-C₆-alcényle, C₃-C₆-halogénealcényle, C₃-C₆-alcynyle, C₃-C₆-halogénealcynyle, X₃R₃₅ ou X₄R₃₆;
R₁₃ signifie l'hydrogène, un groupe C₁-C₆-alkyle, C₃-C₆-alcényle, C₃-C₆-alcynyle ou 1,3-époxy-3-propyle;
R₁₄, R₁₅, R₁₆, R₁₇, R₁₈, R₂₁, R₂₂, R₂₅, R₂₆, R₂₈, R₂₉, R₃₀, R₃₂ et R₃₃ signifient indépendamment les uns des autres l'hydrogène, un groupe C₁-C₄-alkyle, C₃-C₆-alcényle ou C₃-C₆-alcynyle; ou bien R₁₅ et R₂₅ signifient indépendamment l'un de l'autre les groupes -C(O)-X₅-C₁-C₄-alkyle ou -C(O)-C₁-C₄-alkyle, qui de leur côté peuvent être substitués par un halogène; ou bien
R₁₅ et R₁₆ ou R₁₇ et R₁₈ ou R₂₁ et R₂₂ ou R₂₅ et R₂₆ ou R₂₈ et R₂₉ ou R₃₀ et R₁₄ ou R₃₂ et R₃₃ signifient ensemble une chaîne C₄-C₅-alkylène, qui de son côté peut être interrompue par de l'oxygène ou NR₁₉,
R₁₉ signifie l'hydrogène, un groupe C₁-C₄-alkyle, C₃-C₆-alcényle ou C₃-C₆-alcynyle;
R₂₀ et R₃₅ signifient indépendamment l'un de l'autre un groupe C₁-C₄-alkyle ou C₁-C₄-halogènealkyle;
R₂₃ et R₃₆ signifient indépendamment l'un de l'autre un groupe C₁-C₄-alkyle substitué par COOR₃₄;
R₂₄, R₂₇ et R₃₄ signifient indépendamment l'un de l'autre l'hydrogène ou un groupe C₁-C₄-alkyle;
X₁ et X₃ signifient indépendamment l'un de l'autre le soufre, SO ou SO₂;
X₂ et X₄ signifient indépendamment l'un de l'autre l'oxygène ou le soufre;
X₅ signifie l'oxygène ou NR₃₇; et
R₃₇ signifie l'hydrogène, un groupe C₁-C₄-alkyle, C₃-C₆-alcényle ou C₃-C₆-alcynyle;
ou d'un N-oxyde ou d'un sel du composé de formule I, et
b) d'une quantité efficace comme antagoniste du point de vue herbicide d'une sulfamoylphénylurée de formule II 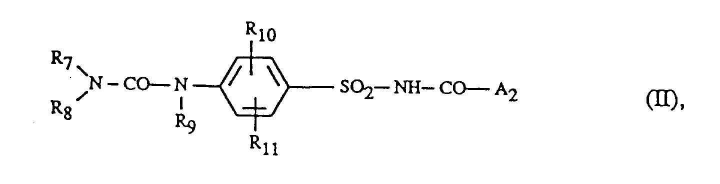 où
A₂ signifie un reste parmi le groupe 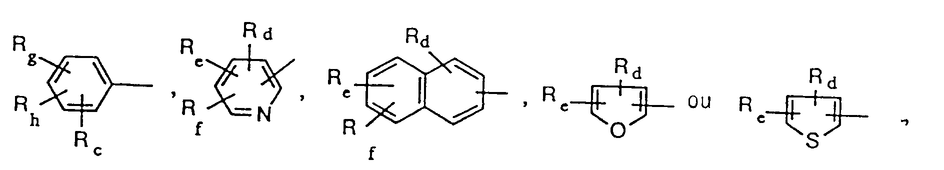
R₇ et R₈ signifient indépendamment l'un de l'autre l'hydrogène, un groupe C₁-C₈-alkyle, C₃-C₈-cycloalkyle, C₃-C₆-alcényle, C₃-C₆-alcynyle, 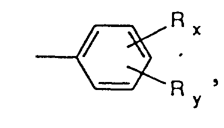 ou signifient un groupe C₁-C₄ alkyle substitué par un groupe C₁-C₄alcoxy ou par 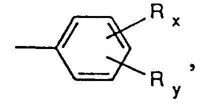 ou
R₇ et R₈ représentent ensemble un pont C₄-C₆-alkylène ou un pont C₄-C₆-alkylène interrompu par de l'oxygène, du soufre, SO, SO₂, NH ou par -N(C₁-C₄-alkyle);
R₉ signifie l'hydrogène ou un groupe C₁-C₄-alkyle;
R₁₀ et R₁₁ signifient indépendamment l'hydrogène, un halogène, un groupe cyano, nitro, trifluorométhyle, C₁-C₄-alkyle, C₁-C₄-alcoxy, C₁-C₄-alkylthio, C₁-C₄-alkylsulfinyle, C₁-C₄-alkylsulfonyle, -COORⱼ, CONRₖRₘ, -CORₙ, -SO₂NRₖRₘ ou -OSO₂-C₁-C₄-alkyle; ou bien
R₁₀ et R₁₁ signifient ensemble un pont C₃-C₄-alkylène qui peut être substitué par un halogène ou un groupe C₁-C₄-alkyle, ou bien un pont C₃-C₄-alcénylène qui peut être substitué par un halogène ou par C₃ ou C₄-alkyle, ou bien un pont butadiénylène qui peut être substitué par un halogène ou un groupe C₁-C₄-alkyle, et
R_{g} et Rₕ signifient indépendamment l'un de l'autre l'hydrogène, un halogène, un groupe C₁-C₄-alkyle, trifluorométhyle, méthoxy, méthylthio ou -COORⱼ, dans ce cas là,
R_{c} signifie l'hydrogène, un halogène, un groupe C₁-C₄-alkyle ou méthoxy;
R_{d} signifie l'hydrogène, un halogène, un groupe nitro, C₁-C₄-alkyle, C₁-C₄-alcoxy, C₁-C₄-alkylthio, C₁-C₄-alkylsulfinyle, C₁-C₄-alkylsulfonyle, -COORⱼ ou -CONRₖRₘ;
Rₑ signifie l'hydrogène, un halogène, un groupe C₁-C₄-halogènealkyle, C₁-C₄-alkyle, -COORⱼ, trifluorométhyle ou méthoxy; ou bien
R_{d} et Rₑ signifient ensemble un pont C₃-C₄-alkylène;
R_{f} signifie l'hydrogène, un halogène ou un groupe C₁-C₄-alkyle;
Rₓ et R_{y} signifient indépendamment l'un de l'autre, l'hydrogène, un halogène, un groupe C₁-C₄-alkyle, C₁-C₄-alcoxy, C₁-C₄-alkylthio, -COOR₁₇, trifluorométhyle, nitro ou cyano;
R₁₇ signifie l'hydrogène, un groupe C₁-C₁₀-alkyle, C₁-C₄-alcoxy-C₁-C₄-alkyle,C₁-C₄-alkylthio-C₁-C₄-alkyle, di-C₁-C₄-alkylamino-C₁-C₄-alkyle, un halogène-C₁-C₈-alkyle, C₂-C₈-alcényle, halogène-C₂-C₈-alcényle, C₃-C₈-alcynyle, C₃-C₇-cycloalkyle, halogène-C₃-C₇-cycloalkyle, C₁-C₈-alkylcarbonyle, allylcarbonyle, C₃-C₇-cycloalkylcarbonyle, benzoyle, non substitué ou substitué sur le cycle phényle de manière identique ou différente jusqu'à 3 fois par un halogène, un groupe C₁-C₄-alkyle, halogène-C₁-C₄-alkyle, halogène-C₁-C₄-alcoxy ou C₁-C₄-alcoxy; ou furoyle, thiényle; ou bien C₁-C₄-alkyle substitué par phényle, halogènephényle, C₁-C₄-alkylphényle, C₁-C₄-alcoxyphényle, halogène- C₁-C₄-alkyl-phényle, halogène-C₁-C₄-alcoxyphényle, C₁-C₆-alcoxycarbonyle, C₁-C₄-alcoxy-C₁-C₈-alcoxycarbonyle, C₃-C₈-alcényloxycarbonyle, C₃-C₈-alcynyloxycarbonyle, C₁-C₈-alkylthiocarbonyle, C₃-C₈-alcénylthiocarbonyle, C₃-C₈-alcynylthiocarbonyle, carbamoyle, mono-C₁-C₄-alkylaminocarbonyle, di-C₁-C₄-alkylaminocarbonyle; ou bien signifie phénylaminocarbonyle qui est non substitué ou substitué sur le cycle phényle de manière identique ou différente jusqu'à 3 fois par un halogène, un groupe C₁-C₄-alkyle, halogène-C₁-C₄-alkyle, halogène-C₁-C₄-alcoxy ou bien un groupe C₁-C₄-alcoxy ou monosubstitué par un groupe cyano ou nitro, ou bien signifie un groupe 1 ,3-dioxolanne-2-yle qui est non substitué ou substitué par un ou deux restes C₁-C₄-alkyle, ou bien signifie un groupe dioxanne-2-yle qui est non substitué ou substitué par un ou deux restes C₁-C₄-alkyle, ou bien signifie un groupe C₁-C₄-alkyle qui est substitué par un groupe cyano, nitro, carboxy ou C₁-C₈-alkylthio-C₁-C₈-alcoxycarbonyle;
Rⱼ, Rₖ et Rₘ signifient indépendamment les uns des autres l'hydrogène ou un groupe C₁-C₄-alkyle;
Rₖ et Rₘ forment ensemble un pont C₄-C₆-alkylène ou un pont C₄-C₆-alkylène interrompu par de l'oxygène, NH ou -N(C₁-C₄-alkyle); et
Rₙ signifie un groupe C₁-C₄-alkyle, phényle ou un groupe phényle substitué par un halogène, un groupe C₁-C₄-alkyle, méthoxy, nitro ou trifluorométhyle;
ou d'un sel du composé de formule II.

2. Un agent selon la revendication 1, caractérisé en ce que R₆ signifie l'hydrogène, un groupe C₁-C₆-alkyle ou C₃-C₆-cycloalkyle.

3. Un agent selon la revendication 1, caractérisé en ce que R₃ signifie un groupe C₁-C₆-alkyle ou C₁-C₆-alkyle qui est mono ou polysubstitué par un halogène, un groupe C₁-C₆-alcoxy, C₃-C₆-alcényloxy, C₃-C₆-alcynyloxy ou C₃-C₆-cycloalkyle qui de son côté peut être interrompu par de l'oxygène, un groupe C₃-C₆-alcényle ou C₃-C₆-alcényle qui est mono- ou polysubstitué par un halogène, un groupe C₁-C₆-alcoxy, C₃-C₆-alcényloxy ou C₃-C₆-alcynyloxy, un groupe C₄-C₆-cycloalkyle ou C₄-C₆-cycloalkyle qui est mono- ou polysubstitué par un halogène ou un groupe C₁-C₆-alcoxy ou peut être interrompu par de l'oxygène, ou bien un groupe C₃-C₆-alcynyle; et
R₆ signifie l'hydrogène ou un groupe C₁-C₆-alkyle.

4. Un agent selon la revendication 1, caractérisé en ce que R₆ signifie l'hydrogène, un groupe C₁-C₆-alkyle, C₃-C₆-cycloalkyle, C₂-C₆-alcynyle; ou bien un groupe C₁-C₆-alkyle ou C₃-C₆-cycloalkyle substitué par un halogène ou un groupe C₁-C₄-alcoxy; un groupe C₂-C₆-alcényle ou C₂-C₆-alcényle substitué par un halogène.

5. Un agent selon la revendication 1, contenant un composé de formule Ia 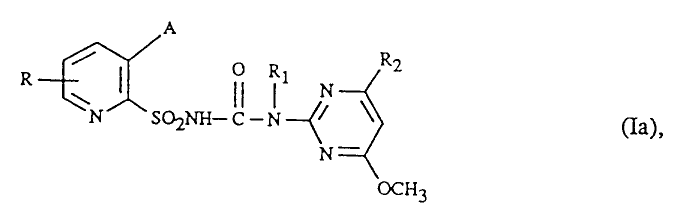 où A, R, R₁, R₂ et R₃ ont les significations indiquées à la revendication 1.

6. Un agent selon la revendication 5, où R signifie l'hydrogène.

7. Un agent selon la revendication 5, où A signifie -X-R₃.

8. Un agent selon la revendication 5, où A signifie -N-(R₄)R₅.

9. Un agent selon la revendication 5, où R₅ signifie C(O)R₆.

10. Un agent selon la revendication 5, où R et R₁ signifient l'hydrogène et A signifie un groupe difluorométhoxy ou le groupe

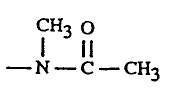

11. Un agent selon la revendication 6, où R et R₁ signifient l'hydrogène et A signifie le groupe 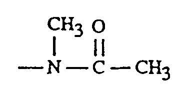 ou -SO₂-C₁-C₄-alkyle.

12. Un agent selon la revendication 1, où dans les composés de formule II
A₂ signifie un reste de formule 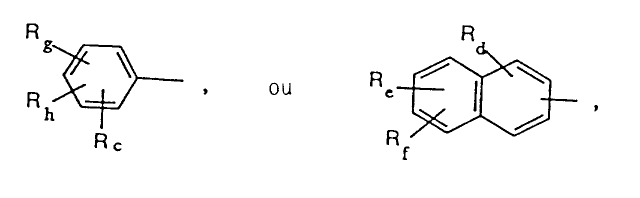 où
R_{g} et Rₕ signifient indépendamment l'un de l'autre l'hydrogène, un halogène, un groupe C₁-C₄-alkyle, trifluorométhyle, méthoxy, méthylthio ou COORⱼ, dans de cas là,
R_{c} signifie l'hydrogène, un halogène, un groupe C₁-C₄-alkyle ou méthoxy,
R_{d} signifie l'hydrogène, un halogène, un groupe nitro, C₁-C₄-alkyle, C₁-C₄-alcoxy, C₁-C₄-alkylthio, C₁-C₄-alkylsulfinyle, C₁-C₄-alkylsulfonyle, -COORⱼ ou -CONRₖRₘ,
Rₑ signifie l'hydrogène, un halogène, un groupe C₁-C₄-alkyle, -COORⱼ, trifluorométhyle ou méthoxy, ou bien R_{d} et Rₑ forment ensemble un pont C₃ ou C₄-alkylène,
R_{f} signifie l'hydrogène, un halogène ou un groupe C₁-C₄-alkyle,
Rⱼ, Rₖ et Rₘ signifient indépendamment les uns des autres, l'hydrogène ou un groupe C₁-C₄-alkyle, et
Rₖ et Rₘ forment ensemble un pont C₄-C₆-alkylène ou un pont C₄-C₆-alkylène interrompu par l'oxygène, NH ou -N(C₁-C₄-alkyle).

13. Un agent selon la revendication 12, où dans les composés de formule II, R_{c}, R_{d}, Rₑ, R_{g} et Rₕ signifient indépendamment les uns des autres, l'hydrogène, un groupe C₁-C₄-alkyle ou méthoxy.

14. Un agent selon la revendication 1, où dans les composés de formule II R₁₀ et R₁₁ signifie l'hydrogène.

15. Un agent selon la revendication 5, contenant un composé de formule IIf 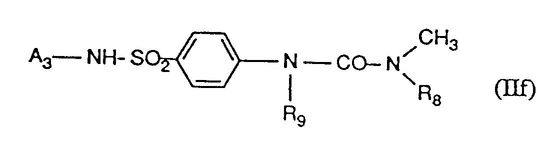 où R₈ et R₉ ont la signification indiquée sous la formule II à la revendication 1, et A₃ signifie les groupes

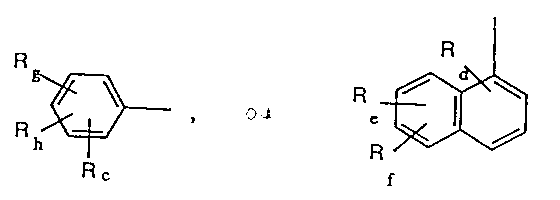

16. Un agent selon la revendication 15, où dans les composés de formule IIf, R₈ et R₉ signifient l'hydrogène ou un groupe méthyle.

17. Un agent selon la revendication 1, où dans les composés de formule II, R₉, R₁₀ et R₁₁ signifient l'hydrogène.

18. Un agent selon la revendication 5, caractérisé en ce que comme composé de formule II, il contient un composé de formule IIa 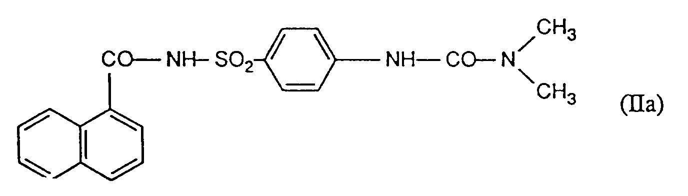 ou de formule IIb 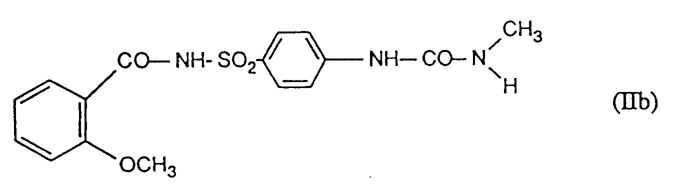 ou de formule IIc 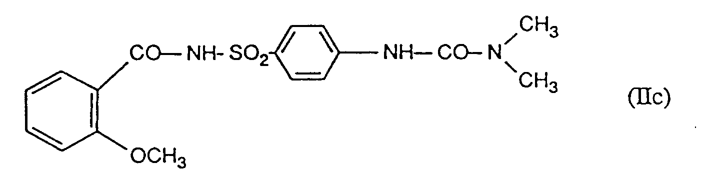 ou de formule IId 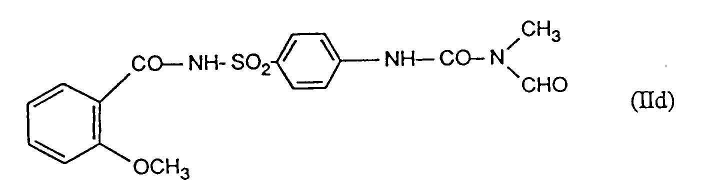 ou de formule IIe

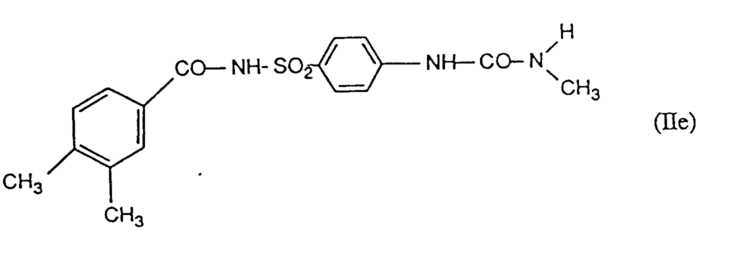

19. Un procédé de lutte sélective contre les mauvaises herbes et les herbes dans les cultures de plantes utiles, caractérisé en ce qu'on traite les cultures, les semences ou les surfaces cultivées avec ces plantes, simultanément ou indépendamment avec une quantité efficace d'un herbicide de formule I selon la revendication 1 et avec une quantité efficace comme antagoniste du point de vue herbicide d'un composé de formule II selon la revendication 1.

20. Un procédé selon la revendication 19, caractérisé en ce qu'on traite les plantes cultivées ou les surfaces cultivées avec ces plantes avec 0,001 à 2 kg/ha d'un composé de formule I selon la revendication 1 et avec une quantité de 0,005 à 0,5 kg/ha d'un composé de formule II selon la revendication 1.

21. Un procédé selon la revendication 19, pour la lutte sélective contre les mauvaises herbes et les herbes dans les cultures de maïs et de canne à sucre.
